# EUROPEAN PATENT SPECIFICATION

(11) **EP 2 590 940 B1**
(45) Date of publication and mention of the grant of the patent: **27.08.2014**
(21) Application number: 11738419.8
(22) Date of filing: 08.07.2011
(51) Int. Cl.: C07D 205/08, C07D 263/38

(54) **NEW METHOD FOR PREPARING EZETIMIBE**
NEUES VERFAHREN ZUR HERSTELLUNG VON EZETIMIB
NOUVEAU PROCÉDÉ DE PRÉPARATION D'ÉZÉTIMIBE

(30) Priority: 09.07.2010 ES 201031052
(43) Date of publication of application: 15.05.2013
(73) Proprietor: Moehs Ibérica, S.L., 08191 Rubí (Barcelona) (ES)
(72) Inventor: SÁNCHEZ CASALS, Carles, E-08191 Rubi-Barcelona (ES); DOBARRO RODRÍGUEZ, Alicia, E-08191 Rubi-Barcelona (ES); BOSCH CARTES, Juan, E-08028 Barcelona (ES); GARCÍA DÍAZ, Davinia, E-08905 Hospitalet de Llobregat-Barcelona (ES)
(74) Representative: ABG Patentes, S.L.
(86) International application number: PCT/EP2011/061616
(87) International publication number: WO 2012/004382

(56) References cited:
- WO-A1-2007/059871
- WO-A2-2010/141494

## Description

### Field of the Invention

The present invention relates to a new method for preparing ezetimibe. The invention also relates to new intermediates used in this method.

### Prior Art

Ezetimibe or 1-(4-fluorophenyl)-3(R)-[3-(4-fluorophenyl)-3(S)-hydroxypropyl]-4(S)-(4-hydroxyphenyl)-2-azetidinone belongs to a class of antihyperlipidemic compounds used for reducing cholesterol levels.

The chemical structure of ezetimibe is represented by:

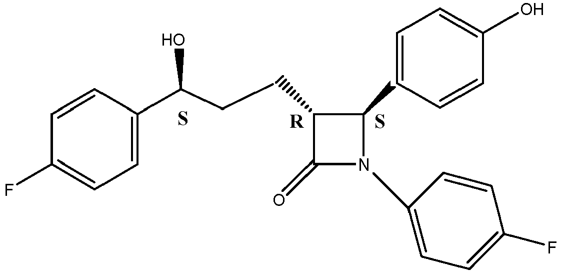

The action of ezetimibe is located in the small intestine inhibiting the absorption of cholesterol from the diet by means of a mechanism that is complementary to that of HMG-CoA reductase enzyme inhibitors. Ezetimibe is marketed in most countries under the name of Zetia (Merck/Schering Plough). The combination of ezetimibe with a statin, specifically simvastatin, is also available on the market.

Ezetimibe was first disclosed in patent application WO 95/08532 A1. The same document also describes a preparation process in 6 synthesis steps, several of the intermediates thereof being obtained in oil form.

Patent application WO 97/45406 A1 discloses a process for preparing ezetimibe comprising the initial reaction between a lactone and an imine to form a chiral diol which is oxidized into the corresponding aldehyde. The preparation of ezetimibe comprises the subsequent use of an enol ether reactant in an aldol condensation, hydrogenation, chiral catalytic reduction and final debenzylation.

Patent application WO 00/34240 A1 discloses a process which comprises reacting the acid (II) with pivaloyl chloride followed by acylation with a chiral auxiliary (III) to obtain an intermediate (IV) 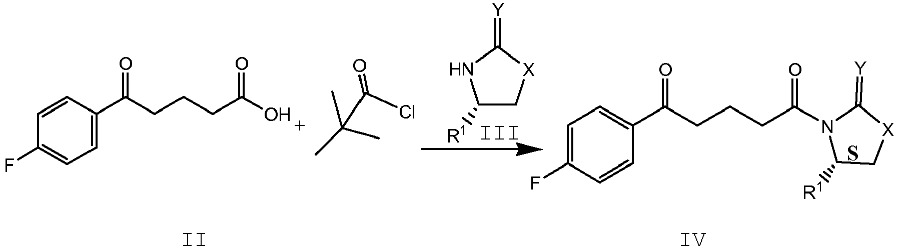 wherein X is -O-, -S- or -N(C₁-C₆ alkyl); Y is =O or =S; and R¹ is C₁-C₆ alkyl, phenyl, naphthyl, substituted phenyl, substituted naphthyl, C₁-C₆ alkoxycarbonyl or benzyl, wherein the phenyl and naphthyl substituents are 1-3 substituents selected from the group consisting of C₁-C₆ alkyl, phenyl and benzyl.

Intermediate IV is then reduced in the presence of a chiral catalyst to obtain the corresponding (S)-alcohol (V) with a 98% diastereoisomeric excess, which is condensed with imine (VI) and a silylated protecting agent which leads to obtaining (VII):

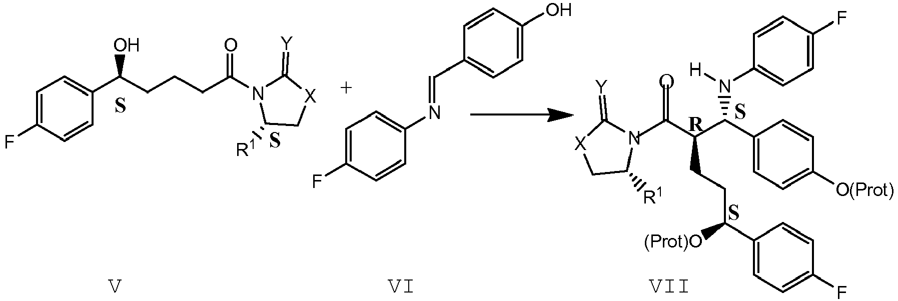

The patent application indicates the stability of the phenolic hydroxyl trimethylsilyl protecting group (Prot) observed throughout the condensation process. However, the diastereoisomeric purity of intermediate VII or the diastereoselectivity observed in the condensation step are not mentioned.

The authors of the present invention have reproduced the example of patent application WO 00/34240 A1, observing a diastereoselectivity in the condensation step of approximately 2.6:1 (SRS:SRR), the virtually pure (diastereosiomerically speaking) condensation product VII being isolated after crystallization.

Cyclization of (VII) and the subsequent deprotection reaction allow obtaining ezetimibe.

Patent applications (WO 2007/072088 A1, WO 2007/119106 A2 or WO 2008/106900 A1) have more recently been published in which processes for obtaining ezetimibe following similar synthesis strategies are described. They are based on protecting the carbonyl group which subsequently gives rise to the ezetimibe 3(S)-hydroxyl group by means of using different acetals. The corresponding addition and cyclization steps are studied in these processes.

Therefore, there is a need in the state of the art for alternative methods for the synthesis of ezetimibe which present improvements with respect to those already existing.

### Summary of the Invention

An object of the present invention is to provide an improved method for the synthesis of ezetimibe in which the first reaction intermediate is obtained with a high diastereoselectivity in relation to that obtained following the methods described in the state of the art.

Another object of the present invention is to provide new intermediates for the synthesis of ezetimibe.

In one aspect of the present invention, a method for preparing ezetimibe (7) comprising the following steps is provided:
(a) reacting a compound of formula (1) with an imine of formula (2) to yield a diastereoisomeric mixture (3a) and (3b) the main product of which is the compound of formula (3a),
(b) optionally hydrolyzing the hydroxyl protecting group to yield a diastereoisomeric mixture (4a) and (4b) the main product of which is the compound of formula (4a),
(c) separating compound (3a) from the diastereoisomeric mixture of step (a), or in the event of having performed step (b), separating compound (4a) from the diastereoisomeric mixture of step (b),
(d) cyclizing the compound of formula (3a) or the compound of formula (4a) with a silylating agent and a fluorine salt to yield compound (5),
(e) reducing and deprotecting compound (5) to yield compound (6),
(f) converting compound (6) into ezetimibe (7) by reaction with a nitrous acid source and subsequent hydrolysis in alkaline aqueous medium.

In another aspect of the invention a compound of formula (3a) is provided.

In another aspect of the invention a compound of formula (3b) is provided.

In yet another aspect of the invention a compound of formula (4a) is provided.

In still another aspect of the invention a compound of formula (4b) is provided.

In another aspect of the invention a compound of formula (5) is provided.

Finally, in another aspect of the invention a compound of formula (6) is provided.

### Detailed Description of the Invention

The authors have developed a method for the synthesis of ezetimibe, the first step of which consists of reacting the compound (4S)-phenyl-3-[(5S)-(4-fluorophenyl)-5-hydroxypentanoyl]-1,3-oxazolidin-2-one protected with a hydroxyl protecting group (formula 1) with the compound of formula (2) 4-fluoro-N-(4-nitrobenzylidene)aniline to give rise to a diastereoisomeric mixture of two products (3a) and (3b), SRS:SRR, at a molar ratio of approximately 8:1, in which the main product, represented by formula (3a), is a new intermediate in the synthesis of ezetimibe. Subsequently, the synthesis continues with the steps of separating compound (3a), cyclizing said compound, reducing and deprotecting the cyclization product and, finally, converting into ezetimibe.

The first step of the synthesis starts from compound (2), in which a nitro group has replaced the hydroxyl group of the starting product used in the methods for the synthesis of ezetimibe known in the state of the art (see, for example, patent application WO 00/34240 A1). Surprisingly, the use of compound (2) with a nitro group rather than the hydroxyl group allows obtaining greater diastereoselectivity in the condensation reaction of compounds (1) and (2), improving the process for producing the final product, ezetimibe.

Specifically, in the experimental example of this invention, a molar ratio of SRS:SRR diastereoisomers of approximately 8:1 is obtained in which the desired diastereoisomer is the main diastereoisomer obtained, whereas in the comparative example conducted by the inventors according to the method described in document WO 00/34240 A1 the ratio is 2.6:1.

In one aspect, the present invention provides a method for preparing ezetimibe (7) comprising the following steps:
(a) reacting a compound of formula (1) 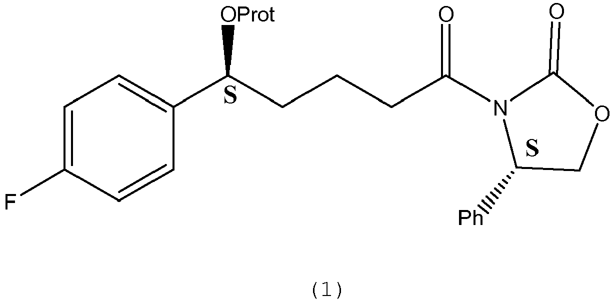 wherein Ph represents a phenyl group and Prot represents a hydroxyl protecting group,
   with an imine of formula (2) 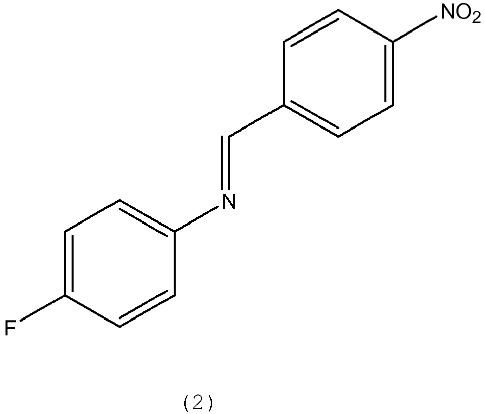 to yield a diastereoisomeric mixture (3a) and (3b) the main product of which is the compound of formula (3a) 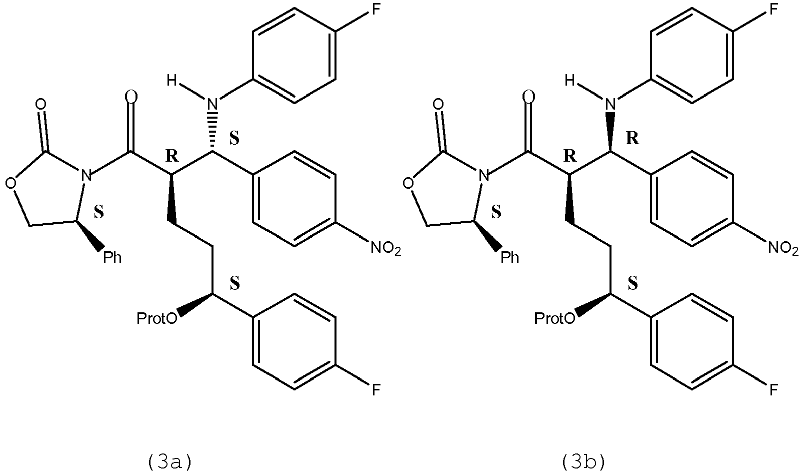
(b) optionally hydrolyzing the hydroxyl protecting group to yield a diastereoisomeric mixture (4a) and (4b) the main product of which is the compound of formula (4a), 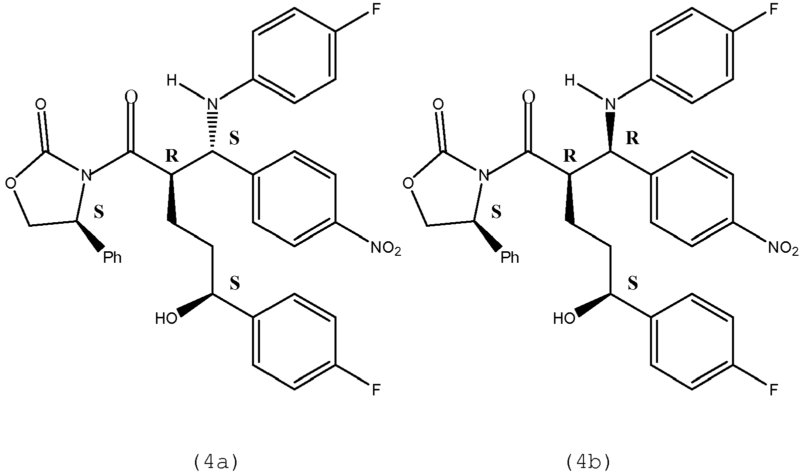
(c) separating compound (3a) from the diastereoisomeric mixture of step (a) or, in the event of having performed step (b), separating compound (4a) from the diastereoisomeric mixture of step (b),
(d) cyclizing the compound of formula (3a) or the compound of formula (4a) with a silylating agent and a fluorine salt to yield compound (5), 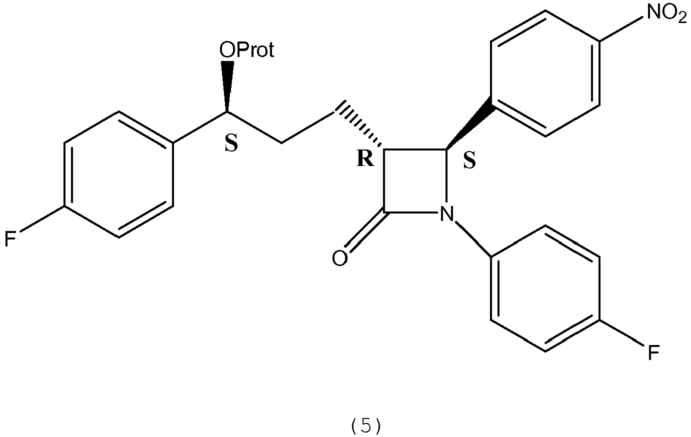 wherein Prot refers to a hydroxyl protecting group, said hydroxyl protecting group being a silyl protecting group if the cyclization is performed from the compound of formula (4a),
(e) reducing and deprotecting compound (5) to yield compound (6), 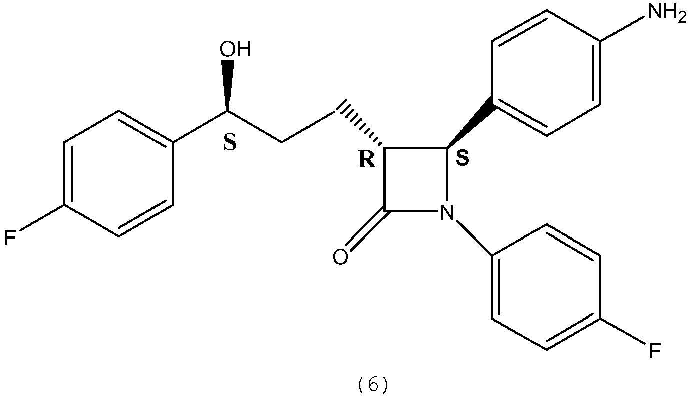
(f) converting compound (6) into ezetimibe (7) by reaction with a nitrous acid source and subsequent hydrolysis in alkaline aqueous medium

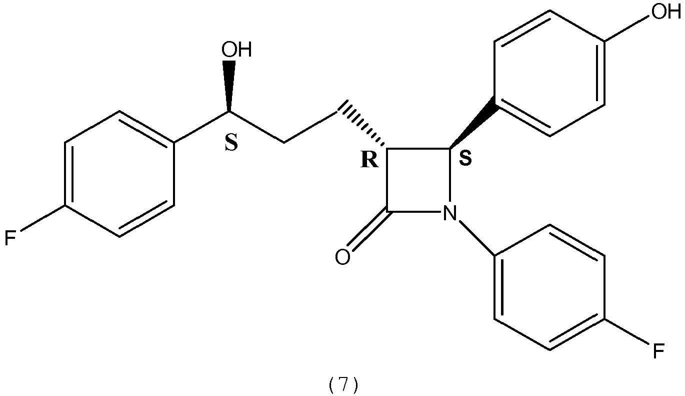

In the first reaction step (step a), the compound (4S)-phenyl-3-[(5S)-(4-fluorophenyl)-5-hydroxypentanoyl]-1,3-oxazolidin-2-one, the hydroxyl of which is protected with a hydroxyl protecting group (1), is subjected to a condensation with the compound 4-fluoro-N-(4-nitrobenzylidene)aniline (2) to obtain a diastereoisomeric mixture (3a) and (3b) in which the main diastereoisomer is (4S)-phenyl-3-[(5S)-(4-fluorophenyl)-(2R)-[(1S)-(4-fluorophenylamino)-1-(4-nitrophenyl)methyl]-5-hydroxypentanoyl]oxazolidin-2-one, the hydroxyl group of which is protected with a hydroxyl protecting group (3a). The reaction is performed in the presence of an anhydrous organic solvent, such as for example CH₂Cl₂ and a Lewis acid such as TiCl₄, optionally in the presence of tertiary amine, such as DIPEA, maintaining the temperature between -20°C and -40°C, more preferably between -35°C and -40°C for a specific time. The reaction is finally stopped by adding an acid, such as glacial acetic acid, followed by an aqueous tartaric acid solution. The addition of glacial acetic acid can produce hydrolysis of the hydroxyl protecting group (optional reaction step b) to yield a diastereoisomeric mixture (4a) and (4b) the main product of which is the compound of formula (4a).

In reaction step (c), the mixture of diastereoisomers obtained in any of the preceding steps is subjected to a separation to obtain the main product (3a) or (4a). The extraction and purification of the main product can be performed by means of conventional techniques such as selective crystallization/precipitation or column chromatography. Therefore in a preferred embodiment the diastereoisomers are separated by means of selective precipitation in ethanol. In another embodiment this separation is a chromatographic separation.

In an embodiment of the present invention, the SRS:SRR diastereoisomeric mixture obtained in step (a) or in step (b) has a molar ratio of both diastereomers greater than 3:1, intermediate (3a) or (4a) being the main diastereoisomer, preferably a molar ratio greater than 4:1, more preferably a molar ratio greater than 5:1, even more preferably a molar ratio greater than 6:1, still more preferably a molar ratio greater than 7:1, most preferably a molar ratio between 7.5:1 and 8.5:1.

The hydroxyl of the compound of formula (1) involved in step (a) has previously been protected with a hydroxyl protecting group, preferably a silyl protecting group, to prevent it from reacting in subsequent reaction steps. In the context of this invention, "hydroxyl protecting group" is understood as a group which cancels out the potential reactivity of the hydroxyl group to favor the chemoselectivity of a subsequent reaction. The hydroxyl group can be protected by means of the formation of ethers (for example, R-O-CH₂-Ph or R-O-CH₃), acetals (R-O-C₅H₁₀O = R-O-tetrahydropyran), esters (R-O-CO-CH₃) or silyl ethers (R-O-SiR₁R₂R₃). In one embodiment of the invention, the hydroxyl protecting group is a silyl protecting group. In the context of this invention, "silyl protecting group" is understood as a group of formula 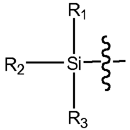 wherein R₁, R₂ and R₃ can be C₁-C₄ alkyl or phenyl. The protecting group can be introduced by means of using a silyl chloride of formula:

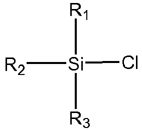

The silyl protecting group is selectively added to the hydroxyl functional group of the compound to be protected, forming a covalent bond between the silicon atom of the protecting group and the oxygen atom of the protected hydroxyl. Silyl chlorides which can be used in the context of the present invention include but are not limited to trimethylsilyl chloride (TMSCl), *tert*-butyldiphenylsilyl chloride (TBDPSCl), *tert*butyldimethylsilyl chloride (TBSCl/TBDMSCl), triisopropylsilyl chloride (TIPSCl), etc. In a preferred embodiment of the invention the silyl protecting group is preferably trimethylsilyl.

Therefore in one embodiment of the invention, compound (1) is obtained from the compound of formula (8) 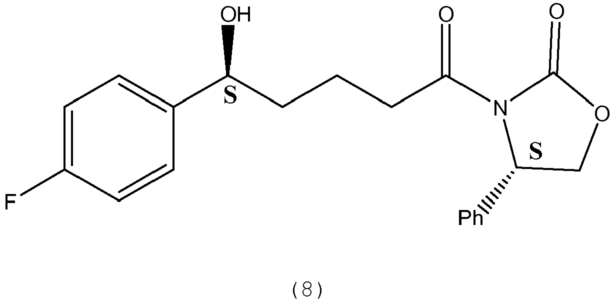 by means of protecting the hydroxyl group with a hydroxyl protecting group.

This reaction is generally carried out with a hydroxyl protecting group and a base in the presence of an anhydrous organic solvent. The base is preferably an amine base. In a particular embodiment, the protection reaction of compound (8) is carried out with trimethylsilyl chloride (TMSCl) and diisopropylethylamine (DIPEA) in the presence of CH₂Cl₂.

When the hydroxyl is protected by means of the formation of an acetal, for example R-O-tetrahydropyran, the protection reaction is performed in acid medium.

"Anhydrous organic solvent" is understood as a chemical substance capable of dissolving non water-soluble substances which does not contain water. Anhydrous organic solvents which can be used in this invention are CH₂Cl₂, toluene, tetrahydrofuran (THF) or 2-methyl-THF, preferably CH₂Cl₂.

"Amine base" is understood as a base containing an amine functional group.

In one embodiment of the present invention, condensation step (a) between the compound of formula (1) and the imine of formula (2) is performed after the protection reaction of the compound of formula (8) to yield the compound of formula (1) without isolating said compound of formula (1).

In one embodiment of the present invention, step (c) is performed after step (a) without performing step (b).

In an alternative embodiment of the present invention, step (b) for hydrolyzing the hydroxyl protecting group is performed between steps (a) and (c).

In reaction step (d), compound (4S)-phenyl-3-[(5S)-(4-fluorophenyl)-(2R)-[(1S)-(4-fluorophenylamino)-1-(4-nitrophenyl)methyl]-5-hydroxypentanoyl]oxazolidin-2-one, the hydroxyl group of which is protected by a hydroxyl protecting group (3a), is cyclized with a silylating agent and a fluorine salt, such as a quaternary ammonium fluoride or an alkaline fluoride, to yield the protected compound (5). In this case, the silylating agent will only act on the secondary amino group of compound (3a), because the hydroxyl group is already protected.

Alternatively, when the diastereoisomeric mixture (3a) and (3b) has been hydrolyzed in step (b) to yield diastereoisomeric mixture (4a) and (4b), compound (4a) is also cyclized during step (d) with a silylating agent and a fluorine salt, such as a quaternary ammonium fluoride or an alkaline fluoride to yield compound (5). In this case, in addition to acting on the secondary amino group of compound (4a), the silylating agent will also protect the hydroxyl group of said compound, which has been deprotected in step (b).

The silylating agent can be N,O-bis-trimethyl silylacetamide, N-methyl-O-trimethylsilylacetamide or iso-propenyloxytrimethylsilane, preferably N,O-bis-trimethyl silylacetamide in a suitable inert organic solvent at a temperature comprised between -20°C and 110°C, preferably between approximately 40°C and 100°C, more preferably between approximately 50°C and 90°C. The silylating agent is capable of causing the silylation of the -NH- group during the first part of step (d), and subsequently the -N-Si- group is selectively desilylated with the fluorine salt, allowing intramolecular cyclization.

The fluorine salt is preferably selected from among quaternary ammonium fluorides and alkaline fluorides. The substituents of the nitrogen atom of the quaternary ammonium fluorides can typically be alkyl and arylalkyl groups. Examples of quaternary ammonium compounds are tetrabutylammonium fluoride and its hydrates. The alkaline fluorides are typically selected from cesium fluoride and potassium fluoride.

When the silylation and the cyclization are performed sequentially, the silylation reaction can continue for three hours at 90°C until adding the fluorine salt, which is preferably carried out at 50°C for more than 2 hours, preferably for 14 hours. The fluoride ion source used to catalyze intramolecular cyclization of the compound of formula (III) is typically a quaternary alkyl fluoride salt, such as tetrabutylammonium fluoride, preferably tetrabutylammonium fluoride trihydrate. The cyclization product can be purified by standard techniques such as crystallization or column chromatography.

The expression "suitable inert organic solvent" refers to any organic solvent or combination of solvents which is not reactive in the reaction being carried out and which is a solvent for the reactants. Suitable solvents are halogenated compounds such as CH₂Cl₂, heterocyclic compounds such as tetrahydrofuran (THF), dimethylsulfoxide (DMSO), dimethylformamide (DMF), acetonitrile, carbocyclic aromatic solvents such as toluene; and t-BuOMe. Toluene is preferred.

In a preferred embodiment of the invention, reaction step (d) is performed with N,O-bis(trimethylsilyl)acetamide, toluene and tetrabutylammonium fluoride.

In the following reaction step (step e), the compound of formula (5) is reduced and deprotected to yield the compound of formula (6). The protecting group is removed using conventional removal methods. When the protecting group is a silyl group, conventional removal methods are used for removing the silyl group, for example by means of the treating with an acid diluted in a solvent. In a preferred embodiment of the invention, step (e) is carried out with Fe/acetic acid, HCl in water and ethanol. The obtained product is extracted and purified by standard techniques, such as crystallization.

The final reaction step (step f) consists of converting the compound of formula (6) into ezetimibe by means of a reaction involving the compound of formula (6) and a nitrous acid source in a suitable solvent, optionally followed by destroying the excess nitrous acid and subsequent hydrolysis in alkaline aqueous medium. Preferably, the nitrous acid source is a nitrous acid salt and an acid, preferably NaNO₂ and an inorganic acid, such as sulfuric acid. "Suitable solvent" is understood as a solvent which allows solubilizing the starting product of formula (6), typically insoluble. Suitable solvents for this solubilization are aprotic polar solvents, such as dimethylformamide (DMF) or dimethylacetamide. The nitrous acid can be destroyed by adding urea. The alkaline aqueous medium in which hydrolysis is performed is preferably a sodium bicarbonate solution.

After adding urea, H₂O can be added to the mixture and it can be basified with the alkaline base. Alternatively, the synthesis can be performed by an inverse addition process, in which after adding urea the obtained mixture is added dropwise to water and, once the addition has ended, the mixture is stirred, cooled and basified with the alkaline base. The final product (ezetimibe) is extracted and purified by standard methods such as crystallization.

In a preferred embodiment of the invention, the nitrous acid source of step (f) is NaNO₂ and sulfuric acid; and the alkaline aqueous medium is a sodium bicarbonate solution.

In a preferred embodiment of the invention, there is an additional step (step b) between steps (a) and (c) in which the hydroxyl protecting group is hydrolyzed.

The intermediates (3a), (3b), (4a), (4b), (5) and (6) obtained in the synthesis reaction of ezetimibe of the invention have not been previously described.

Therefore, another aspect of the invention is to provide an intermediate compound of formula (3a) wherein Prot is a hydroxyl protecting group, preferably a silyl protecting group, more preferably it is trimethylsilyl.

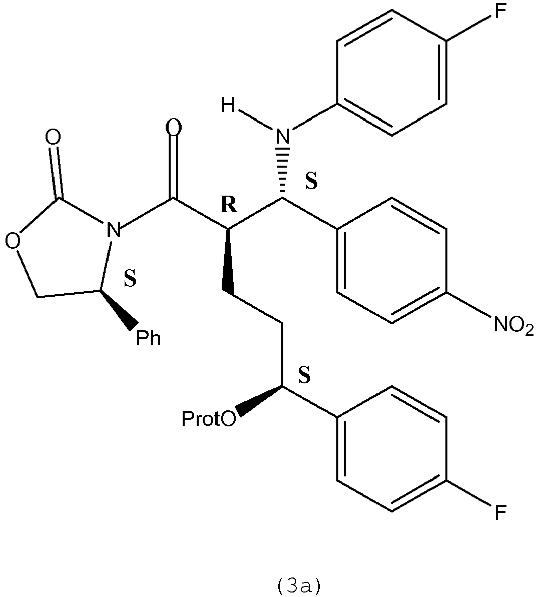

Another aspect of the invention is to provide an intermediate compound of formula (3b) wherein Prot is a hydroxyl protecting group, preferably a silyl protecting group, more preferably it is trimethylsilyl.

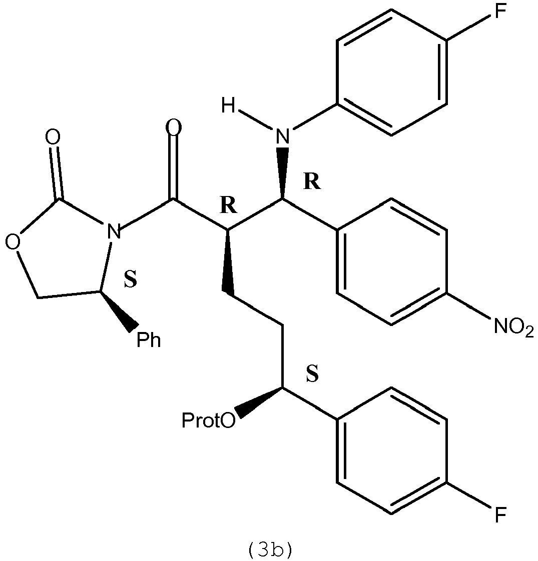

In still another aspect of the invention, an intermediate compound of formula (4a) is provided

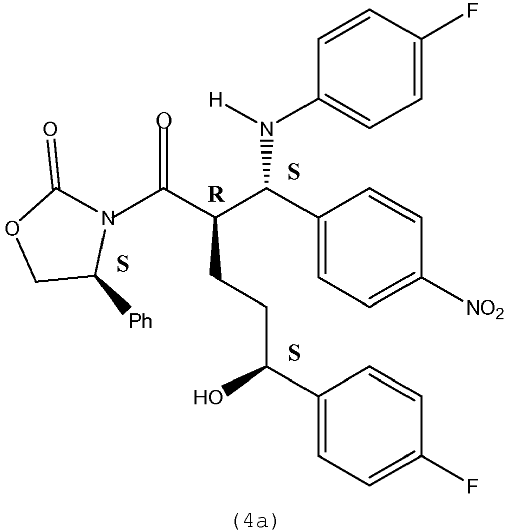

In still another aspect of the invention, an intermediate compound of formula (4b) is provided

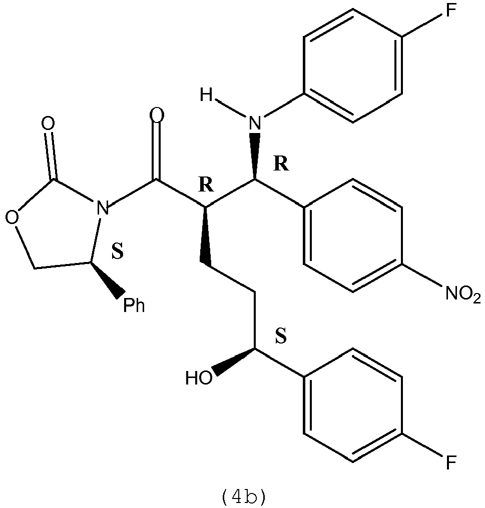

In yet another aspect of the invention, an intermediate compound of formula (5) is provided wherein Prot is a hydroxyl protecting group, preferably a silyl protecting group, more preferably trimethylsilyl.

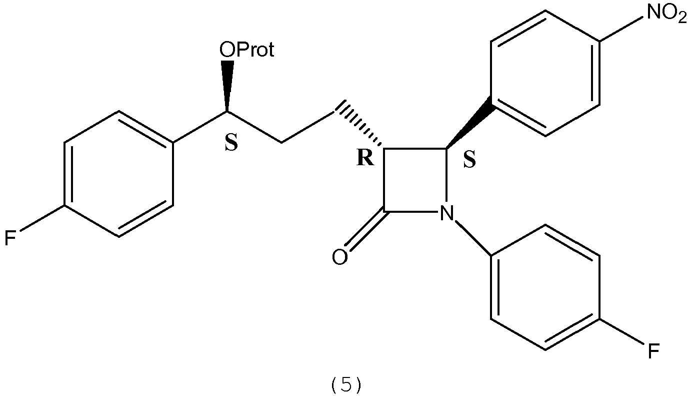

In a final aspect of the invention an intermediate compound of formula (6) is provided.

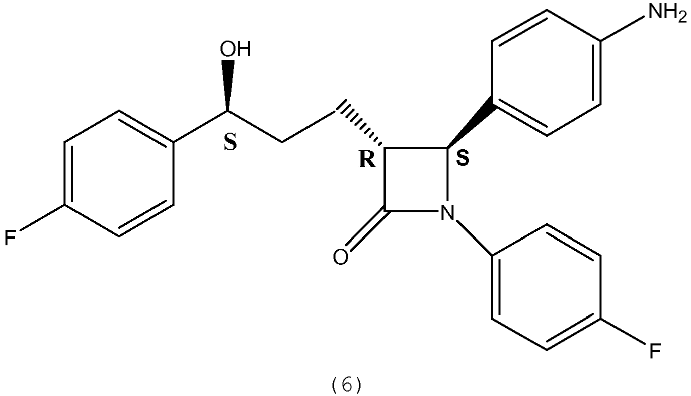

The embodiments included in the present specification describe in detail suitable processes for the synthesis of ezetimibe according to the method of the invention. It is obvious for the person skilled in the art that these examples are only illustrative and should not be considered as being a limitation to the scope of the invention.

### Examples

The compounds obtained in the examples described below are identified by proton (¹H-NMR) and carbon-13 (¹³C-NMR) nuclear magnetic resonance data.

The operating frequency and the solvent used for recording the spectrum are indicated in the spectra. The positions of the signals are indicated in δ (ppm) and the signal of the protons of the solvent are used as a reference. The coupling constants (J) are expressed in Hertz. The number of protons of each signal measured by electron integration and the type of signal are indicated in parenthesis, the latter being indicated using the following abbreviations: s (singlet), d (doublet), t (triplet), q (quadruplet), dd (doublet of doublets), ddd (doublet of doublet of doublets), td (triplet of doublets), m (multiplet), br (broad signal).

The following abbreviations are used in the experimental section:
COSY: proton homonuclear correlation spectroscopy
HSQC: heteronuclear single quantum coherence spectroscopy
EA: Elemental analysis
OTMS: trimethylsilyl ether
TBAF: tetrabutylammonium fluoride
AcOEt: ethyl acetate
DMF: dimethylformamide
DIPEA: diisopropylethylamine
TMSCl: trimethylsilyl chloride

### Example 1: Preparation of the compound of formula (1)

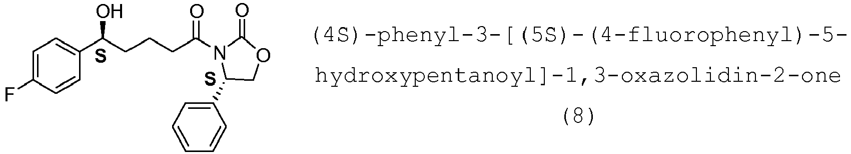

Spectroscopic data of the compound of formula (8):
¹H-NMR (400 MHz, CDCl₃, COSY, HSQC): δ (ppm) 1.60 - 1.77 (m, 4H, CHOHC*H*₂C*H*₂), 2.02 (br s, 1H, OH), 2.97 (t, *J=* 6.4 Hz, 2H, C*H*₂CO), 4.27 (dd, *J=* 8.8, 3.6 Hz, 1H, C*H*₂OCO), 4.63 (dd, *J*= 7.0, 5.0 Hz, C*H*OH), 4.67 (dd, *J=* 8.8, 8.8 Hz, 1H, C*H*₂OCO), 5.40 (dd, *J*= 8.8, 3.6 Hz, 1H, CHN), 7.00 (dd, *J=* 8.4, 8.4 Hz, 2H-Ar, *m*-PhF), 7.24 - 7.29 (m, 4H-Ar), 7.33 - 7.39 (m, 3H-Ar).
¹³C-NMR (100.4 MHz, CDCl₃, HSQC) : δ (ppm) 20.2 (CHOHCH₂CH₂), 35.2 (CH₂CO), 38.2 (CHOH*C*H₂), 57.6 (CHN), 70.0(*C*H₂OCO , 73.3 (CHOH), 115.2 (d, *J=* 21.0 Hz, CH, m-PhF), 125.9 (CH, o-Ph), 127.4 (d, *J=* 7.7 Hz, CH, o-PhF), 128.8 (CH, p-Ph), 129.2 (CH, m-Ph), 139.1 (C, *ipso-Ph),* 140.2 (d, *J=* 3.1 Hz, C, *ipso-PhF),* 153.7 (NCOO), 162.1 (d, *J=* 244.6 Hz, C, p-PhF), 172.5 (CH₂CON).
EA calculated for C₂₀H₂₀FNO₄: C, 67.22%; H, 5.64%; N, 3.92%. Found: C, 67.33%; H, 5.76%; N, 3.88%.

Diisopropylethylamine (39 ml, 223.8 mmol) and trimethylsilyl chloride (13.4 ml, 105 mmol) were added dropwise to a solution of compound 8 (25 g, 69.95 mmol) in CH₂Cl₂ (250 ml) at -10°C until completing the protection of the alcohol to obtain compound 1.

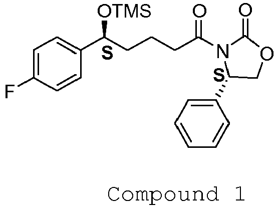

### Example 2: Preparation of the compound of formula (2)

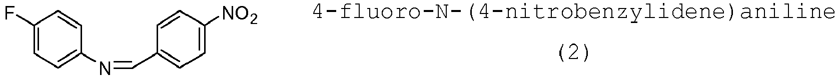

4-Fluoroaniline (17.2 ml, 179.3 mmol) was added to a solution of 4-nitrobenzaldehyde (27.1 g, 179.3 mmol) in methanol (600 ml). The obtained mixture was stirred at room temperature for 2.5 hours and was then evaporated to dryness yielding 2 (43.36 g) as a yellow solid.

Spectroscopic data of the compound of formula (2):
¹H-NMR (300 MHz, CDCl₃): δ (ppm) 7.11 (dd, *J=* 8.7, 8.7 Hz, 2H, *m-*PhF), 7.27 (dd, *J*= 8.7, 4.8 Hz, 2H, *o*-PhF), 8.06 (d, *J=* 9.0 Hz, 2H, *o*-PhNO₂), 8.31 (d, *J=* 9.0 Hz, 2H, m-PhNO₂), 8.54 (s, 1H, CH=N).
¹³C-NMR (75.4 MHz, CDCl₃): δ (ppm) 116.1 (d, *J=* 22.4 Hz, CH, *m-*PhF), 122.6 (d, *J=* 8.6 Hz, CH, o-PhF), 123.9 (CH, *m*-PhNO₂), 129.3 (CH, *o*-PhNO₂), 141.4 (C, *ipso-*PhNO₂)*,* 146.8 (d, *J=* 3.5 Hz, C, *ipso*-PhF)*,* 149.2 (C, *p*-PhNO₂), 156.9 (CH=N), 161.8 (d, *J=* 246.3 Hz, C, p-PhF).
EA calculated for C₁₃H₉FN₂O₂: C, 63.93%; H, 3.71%; N, 11.47%. Found: C, 63.72%, H, 3.90%; N, 11.56%.

### Example 3: Condensation reaction of the compounds of formula (1) and (2). Reaction steps (a), (b) and (c).

Once the protection of the alcohol was completed, the temperature was reduced to -40°C, TiCl₄ (9.2 ml, 84 mmol) was added dropwise to the solution obtained in Example 1 for 45 minutes and then a solution of 2 (11.65 g, 45.45 mmol) in CH₂Cl₂ (100 ml) was added for 30 minutes, maintaining the temperature between -35°C and -40°C. After 2 hours at the same temperature a solution of 2 (11.65 g, 45.45 mmol) in CH₂Cl₂ (100 ml) was added again. Stirring was maintained at -38°C for another 17 hours. The crude reaction product shows a ratio of SRS:SRR diastereoisomers (3a) and (3b) of 8:1 by means of ¹H-NMR analysis.

Glacial acetic acid (20 ml) was added after the maintenance time elapsed. The obtained mixture was stirred for 1 hour, allowing the temperature to increase, was poured onto 7% tartaric acid (500 ml) at 0°C and was stirred for another hour. 20% NaHSO₃ (250 ml) was then added at room temperature and it was stirred again for 1 hour. The organic phase was separated and the aqueous phase was extracted with CH₂Cl₂ (200 ml). The organic phases were washed with brine (2 x 200 ml), dried with MgSO₄, filtered and evaporated. The obtained crude product (mixture of diastereoisomers 4a and 4b) was purified by precipitation in ethanol, yielding 4a (32.9 g) as a slightly yellowish solid.

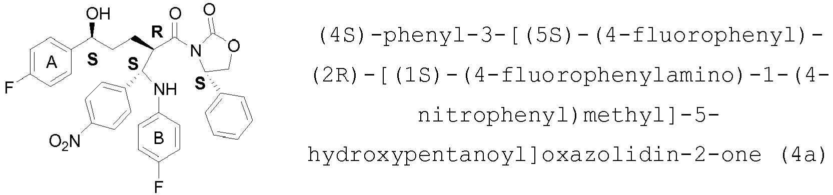

Spectroscopic data of the compound of formula (4a):
¹H-NMR (400 MHz, CDCl₃, HSQC, COSY): δ (ppm) 1.59 - 1.69 (m, 2H, C*H*₂CHOH), 1.75 - 1.82 (m, 2H, C*H*₂CHCO), 4.21 (dd, *J=* 8.8, 3.2 Hz, 1H, CH₂O), 4.51 (d, *J=* 7.2 Hz, 1H, CHNH), 4.58 - 4.61 (m, 2H, CHCO, C*H*OH), 4.64 (dd, *J=* 8.8, Hz, 1H, CH₂O), 5.38 (dd, *J=* 8.8, 3.2 Hz, 1H, NCHPh), 6.34 (dd, *J=* 8.8, 8.4 Hz, 2H, *o*-PHF^{B}), 6.75 (dd, *J=* 8.8, 8.8 Hz, 2H, *m*-PhF^{B}) , 6.98 - 7.02 (m, 4H, *m-*PhF^{A}, o-Ph), 7.10 (dd, *J*= 7.6, 7.6 Hz, 2H, *m*-Ph)*,* 7.20 - 7.25 (m, 3H, *o*-PhF^{A}, *p*-Ph), 7.29 (d, *J*= 8.8 Hz, 2H, *o*-PhNO₂), 8.00 (d, *J=* 8.8 Hz, 2H, *m*-PhNO₂) .
¹³C-NMR (100.4 MHz, CDCl₃): δ (ppm) 26.7 (CH₂CHCO), 36.1 (*C*H₂CHOH), 47.3 (CHCO), 57.8 (NCHPh), 60.2 (CHNH), 70.0 (*C*H₂OCO), 73.3 (CHOH), 114.7 (d, *J=* 6.9 Hz, CH, *o*-PhF^{B}), 115.4 (d, *J=* 21.8 Hz, CH, *m*-PhF^{A}), 115.7 (d, *J=* 22.6 Hz, CH, *m*-PhF^{B}), 123.9 (CH, *m-*PhNO₂), 125.6 (CH, *o*-Ph), 127.4 (d, *J=* 8.6 Hz, CH, *o*-PhF^{A}), 127.8 (CH, *o*-PhNO₂), 128.6 (CH, p-Ph), 129.0 (CH, m-Ph), 138.1 (C, *ipso-Ph),* 139.7 (d, *J=* 3.1 Hz, C, *ipso*-PhF^{A}), 142.0 (C, *ipso-*PhF^{B}), 147.2 (C-PhNO₂), 148.1 (C-PhNO₂), 154.0 (NCOO), 156.1 (d, *J=* 236.3 Hz, C, *p*-PhF^{B}), 162.2 (d, *J=* 245.6 Hz, C, *p*-PhF^{A}), 174.2 (CHCO).
EA calculated for C₃₃H₂₉F₂N₃O₆: C, 65.88%; H, 4.86%; N, 6.98%. Found: C, 65.84%; H, 4.97%; N, 6.94%

### Example 4: Silylation and cyclization reaction of the compound of formula (4a). Reaction step (d).

N,O-bis-trimethylsilylacetamide (5.6 ml, 22.9 mmol) was added to a solution of 4a (3.45 g, 5.73 mmol) in anhydrous toluene (38 ml) at 90°C. After stirring for 3 hours at 90°C the temperature was reduced to 50°C, TBAF·3H₂O (271 mg, 0.86 mmol) was added and it was stirred at the same temperature for 14 hours. After this time elapsed, the mixture was cooled, acetic acid (0.2 ml) was added and it was evaporated to dryness. The obtained crude product was purified by column chromatography (SiO₂) with hexane:AcOEt 9:1 yielding compound 5 (1.76 g).

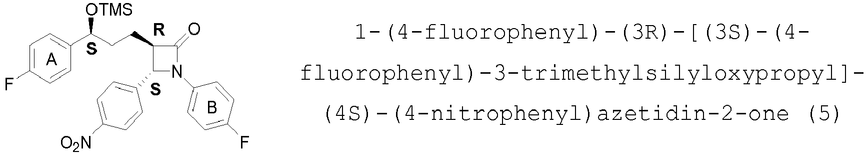

Spectroscopic data of the compound of formula (5):
¹H-NMR (400MHz, CDCl₃, HSQC, COSY): δ (ppm) 0.00 (s, 9H, OTMS), 1.79 - 1.84 (m, 2H, C*H*₂CHOTMS), 1 . 90 - 1.97 (m, 2H, C*H*₂CHCO), 3.05 (td, *J=* 7.6, 2.4 Hz, 1H, CHCO), 4.65 (t, *J=* 6.0 Hz, 1H, CHOTMS), 4.72 (d, *J=* 2.4 Hz, 1H, CHN), 6.92 - 7.01 (m, 4H, *m-*PhF^{A}, *m*-PhF^{B}) , 7.16 - 7.19 (m, 2H, *o*-PhF^{B}), 7.21 - 7.25 (m, 2H, *o*-PhF^{A}), 7.47 (d, *J=* 8.8 Hz, 2H, *o*-PhNO₂), 8.22 (d, *J=* 8.8 Hz, 2H, *m*-PhNO₂).
¹³C-NMR (100.4 MHz, CDCl₃): δ (ppm) -0.01 (CH₃, OTMS), 25.1 (*C*H₂CHCO), 37.9 (CH₂CHOTMS), 60.3 (CHN), 60.9 (CHCO), 73.8 (CHOTMS), 115.1 (d, *J=* 21.8 Hz, CH, *m*-PhF^{A}), 116.1 (d, *J=* 22.5 δ Hz, CH, *m*-PhF^{B}), 118.1 (d, *J=* 7.7 Hz, CH, *o*-PhF^{B}), 124.6 (CH, *m-*PhNO₂), 126.6 (CH, *o*-PhNO₂), 127.2 (d, *J=* 7.7 Hz, CH, *o*-PhF^{A}), 133.4 (d, *J=* 2.4 Hz, C, *ipso*-PhF^{B}), 140.3 (d, *J=* 3.1 Hz, C, *ipso*-PhF^{A})*,* 145.1 (C, *ipso-*PhNO₂*),* 148.0 (C, *p*-PhNO₂), 159.1 (d, *J=* 244.1 Hz, C, *p*-PhF^{B}), 161.9 (d, *J=* 244.9 Hz, C, *p*-PhF^{A}), 166.3 (CO) .

### Example 5: Deprotection and reduction reaction of the compound of formula (5). Reaction step (e).

Acetic acid (17 ml), H₂O (3.3 ml), 6N HCl (0.33 ml) and Fe (2.49 g, 44.55 mmol) were added consecutively to a solution of 5 (3.25 g, 6.36 mmol) in ethanol (17 ml) at 0°C. The obtained mixture was stirred at room temperature for 30 minutes. After this time elapsed H₂O (5 ml) was added and it was filtered through Celite^{®}. The filtrate was extracted with CH₂Cl₂ (2 x 30 ml) and the organic phases were washed with saturated solution of NaHCO₃ (40 ml), dried with MgSO₄, filtered and evaporated. The obtained crude product was purified by crystallization in hexane/AcOEt yielding 6 (2.2 g).

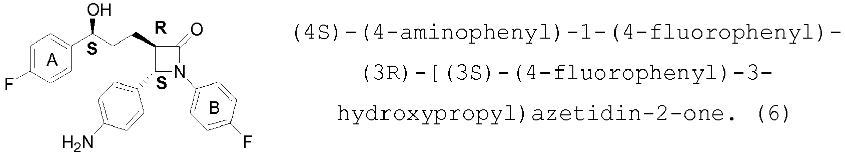

Spectroscopic data of the compound of formula (6):
¹H-NMR (400 MHz, CDCl₃, HSQC, COSY): δ (ppm) 1.86 - 1.99 (m, 4H, CH₂CH₂), 3.05 (td, *J=* 7.4, 2.4 Hz, 1H, CHCO), 4.50 (d, *J=* 2.4 Hz, 1H, CHN), 4.66 - 4.69 (m, 1H, CHOH) 6.64 (d, *J=* 8.4 Hz, 2H, *m-*PhNH₂), 6.90 (dd, J= 8.6, 8.6 Hz, 2H, *m*-PhF^{B}) , 6.99 (dd, *J=* 8.6, 8.6 Hz, 2H, *m*-PhF^{A}), 7.09 (d, *J=* 8.4 Hz, 2H, *o*-PhNH₂), 7.21 - 7.28 (m, 4H-Ar).
¹³C-NMR (100.4 MHz, CDCl₃): δ (ppm) 24.9 (CH₂CHCO), 36.6 (*C*H₂CHOH), 60.1 (CHCO), 61.3 (CHN), 72.9 (CHOH), 115.2 (d, *J=* 21.4 Hz, *m*-PhF^{A}), 115.5 (CH, *m*-PhNH₂), 115.7 (d, *J=* 22.9 Hz, *m-*PhF^{B}), 118.4 (d, *J=* 7.6 Hz, *o*-PhF^{B}), 126.8 (C, *ipso-*PhNH₂)*,* 127.1 (CH, *o*-PhNH₂), 127.3 (d, *J=* 7.6 Hz, *o*-PhF^{A}), 133.9 (C, *ipso-*PhF^{B}), 140.1 (C, *ipso*-PhF^{A})*,* 146.8 (C, *p*-PhNH₂), 158.9 (d, *J=* 243.3 Hz, C, *p*-PhF^{B}), 162.1 (d, *J=* 244.1 Hz, C, *p*-PhF^{A}), 167.9 (CO).

### Example 6: Conversion of the compound of formula (6) into ezetimibe (7). Reaction step (f).

A solution of H₂SO₄ (0.16 ml, 2.94 mmol in 8 ml H₂O) and immediately thereafter an aqueous solution (5 ml) of NaNO₂ (81 mg, 1.17 mmol) were added dropwise to a solution of 6 (400 mg, 0.98 mmol) in DMF (5 ml) at 0°C. The mixture was stirred at 0°C for 1.5 hours. Urea (15 mg, 0.245 mmol) was then added and it was stirred at room temperature for 10 minutes and finally at 90°C until N₂ was no longer generated (approximately 15 minutes). H₂O (5 ml) was then added, it was basified with NaHCO₃ and extracted with AcOEt (3 x 15 ml). The organic phases were concentrated and washed with brine (4 x 20 ml) to remove the DMF, dried with MgSO₄ and filtered. The product was finally crystallized by adding hexane, yielding ezetimibe, 7 (328 mg), as a white solid.

### Inverse addition process:

A solution of H₂SO₄ (0.13 ml, 2.5 mmol in 7 ml H₂O) and immediately thereafter an aqueous solution (4 ml) of NaNO₂ (69 mg, 1 mmol) were added dropwise to a solution of 6 (340 mg, 0.83 mmol) in DMF (4.2 ml) at 0°C. The mixture was stirred at 0°C for 1.5 hours. Urea (13 mg, 0.2 mmol) was then added and the obtained mixture was added dropwise for 10 minutes to 15 ml of H₂O at 85°C, watching to make sure that the temperature did not drop below 80°C. Once the addition was completed, the mixture was stirred another 10 minutes, cooled, basified with NaHCO₃ and extracted with AcOEt (3 x 20 ml). The organic phases were concentrated and washed with brine (4 x 25 ml), dried with MgSO₄ and filtered, and the product was crystallized by adding heptane, yielding 7 (275 mg) as a white solid.

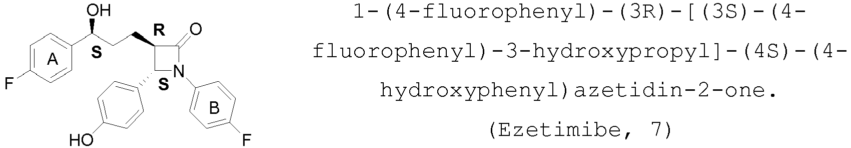

Spectroscopic data of ezetimibe (7):
¹H-NMR (400 MHz, CDCl₃): δ (ppm) 1.85 - 2.01 (m, 4H, CH₂CH₂), 3.05 - 3.09 (m, 1H, CHCO), 4.56 (d, *J=* 2.4 Hz, 1H, CHN), 4.71 (t, J= 5.8 Hz, 1H, C*H*OH), 6.83 (d, *J=* 8.4 Hz, 2H, *m*-PhOH), 6.92 (dd, *J=* 8.8, 8.8 Hz, 2H, *m*-PhF^{B}), 7.01 (dd, *J=* 8.8, 8.8 Hz, 2H, m-PhF^{A}), 7.19 (d, *J=* 8.4 Hz, 2H-Ar), 7.21 - 7.25 (m, 2H, *o*-PhF^{B}), 7.27 - 7.31 (m, 2H-Ar).
¹³C-NMR (100.4 MHz, CDCl₃): δ (ppm) 25.0 (*C*H₂CHCO), 36.5 (*C*H₂CHOH), 60.2 (CHCO), 61.2 (CHN), 73.1 (CHOH), 115.4 (d, *J=* 21.0 Hz, CH, *m*-PhF^{A}), 115.8 (d, *J=* 23.3 Hz, CH, *m*-PhF^{B}), 116.1 (CH, *m*-PhOH), 118.4 (d, *J=* 7.7 Hz, CH, *o*-PhF^{B}), 127.3 (CH-Ar), 127.4 (CH-Ar), 133.8 (C, *ipso*-PhF^{B}), 139.9 (d, *J=* 3.1 Hz, C, *ipso-*PhF^{A}), 156.1 (C, p-PhOH), 159.0 (d, *J=* 243.3 Hz, C, *p*-PhF^{B}), 162.2 (d, *J=* 245.6 Hz, C, *p*-PhF^{A}), 167.8 (CO).

### Comparative example: Condensation reaction of the compounds of formula (V) and (VI) of document WO 00/34240 A1.

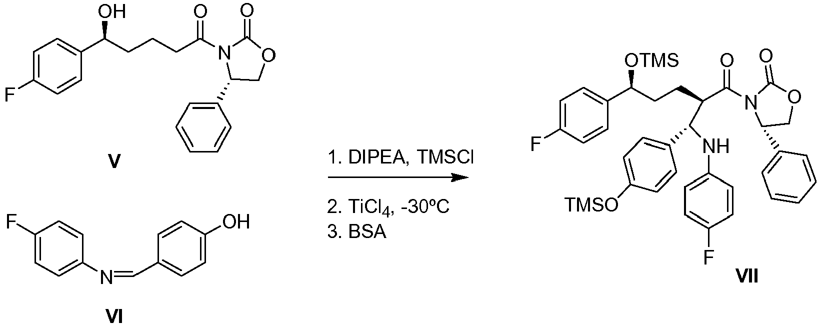

DIPEA (2.56 ml, 14.7 mmol) and TMSC1 (1.17 ml, 9.24 mmol) were added dropwise to a solution of V (1 g, 2.8 mmol) and VI (1.2 g, 5.29 mmol) in anhydrous CH₂Cl₂ (16 ml) at -10 ° C, maintaining the temperature below -5°C. Once the protection was completed(1 hour), the temperature was reduced to -30°C, TiCl₄ (0.34 ml, 3.08 mmol) was added dropwise and stirring was maintained at -30°C for 20 hours. (Starting product was observed after 4 hours by thin layer chromatography). The ¹H-NMR of the obtained crude reaction product shows a 2.6:1 mixture of stereoisomers.

Glacial acetic acid (0.8 ml) was added after this time elapsed. The obtained mixture was stirred for 30 minutes, allowing the temperature to increase, was poured onto tartaric acid 7% (14 ml) at 0°C and stirred for 1 hour, allowing the temperature to increase. 20% NaHSO₃ (5 ml) was then added and it was stirred for another hour. The organic phase was separated, washed with H₂O (15 ml), dried with MgSO₄, filtered and concentrated to an approximate volume of 10 ml. The obtained solution was treated with bistrimethylsilyl acetamide (0.82 ml, 3.36 mmol) under reflux for 30 minutes and evaporated to dryness.

The main stereoisomer was purified by crystallization in AcOEt:heptane (6:4), yielding VII (1.21 g, 60%).

## Claims

1. A method for preparing ezetimibe (7) comprising the following steps:
(a) reacting a compound of formula (1) 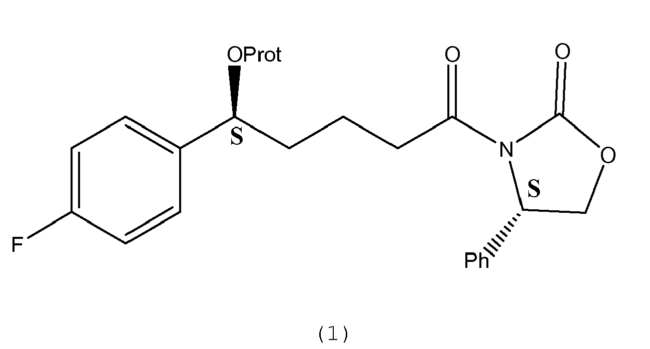 wherein Ph represents a phenyl group and Prot represents a hydroxyl protecting group,
with an imine of formula (2) 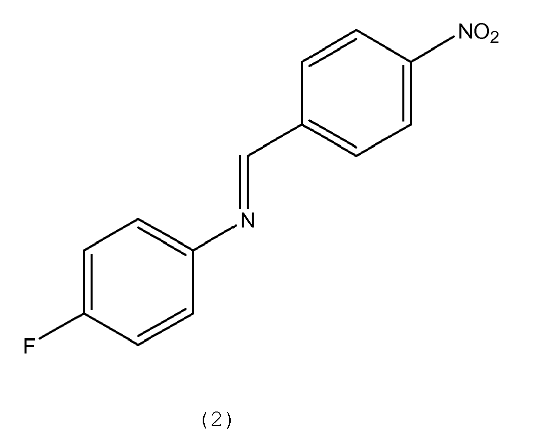 to yield a diastereoisomeric mixture (3a) and (3b) the main product of which is the compound of formula (3a), 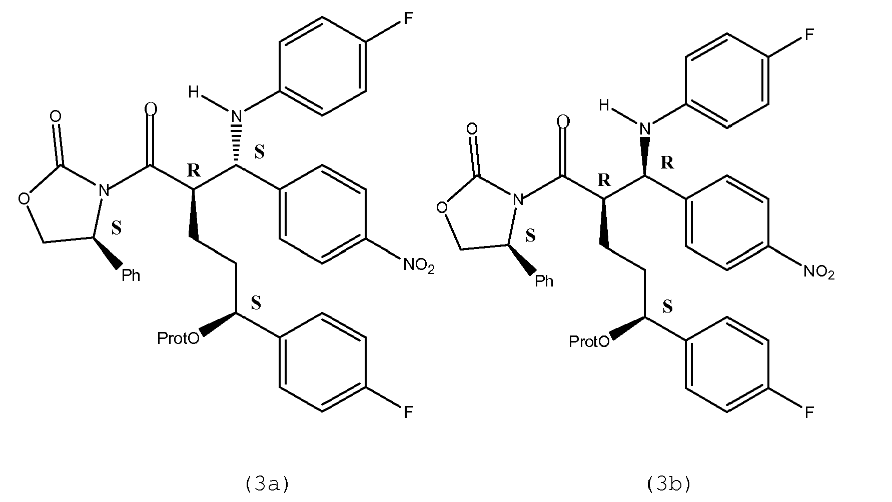
(b) optionally hydrolyzing the hydroxyl protecting group to yield a diastereoisomeric mixture (4a) and (4b) the main product of which is the compound of formula (4a), 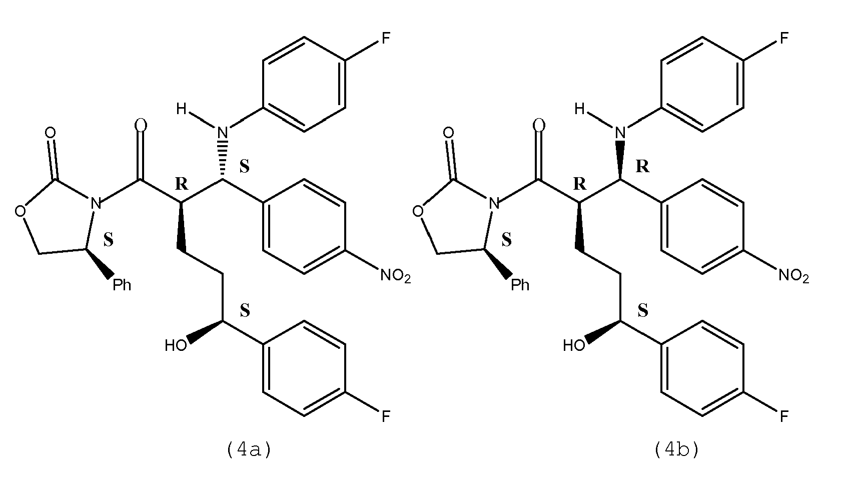
(c) separating compound (3a) from the diastereoisomeric mixture of step (a) or, in the event of having performed step (b), separating compound (4a) from the diastereoisomeric mixture of step (b),
(d) cyclizing the compound of formula (3a) or the compound of formula (4a) with a silylating agent and a fluorine salt to yield compound (5), 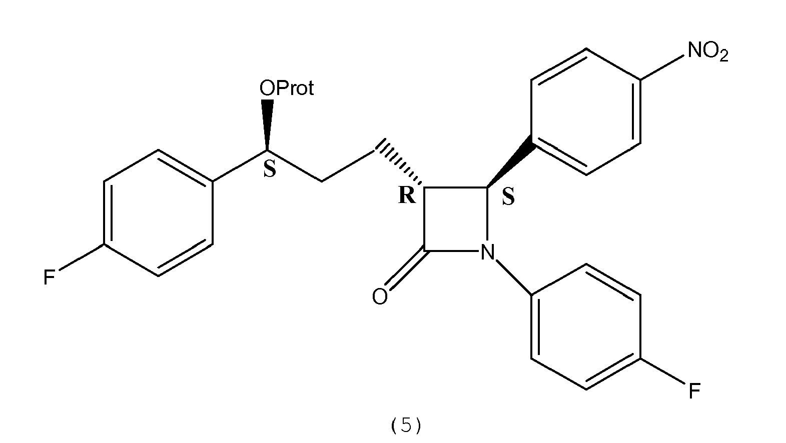
(e) reducing and deprotecting compound (5) to yield compound (6), 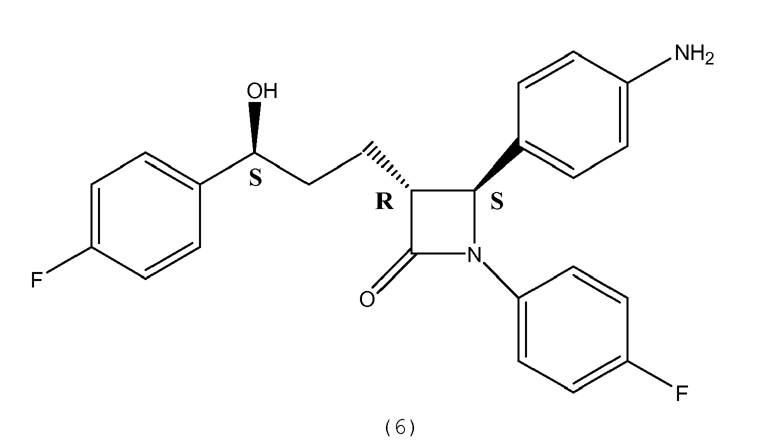
(f) converting compound (6) into ezetimibe (7) by reaction with a nitrous acid source and subsequent hydrolysis in alkaline aqueous medium

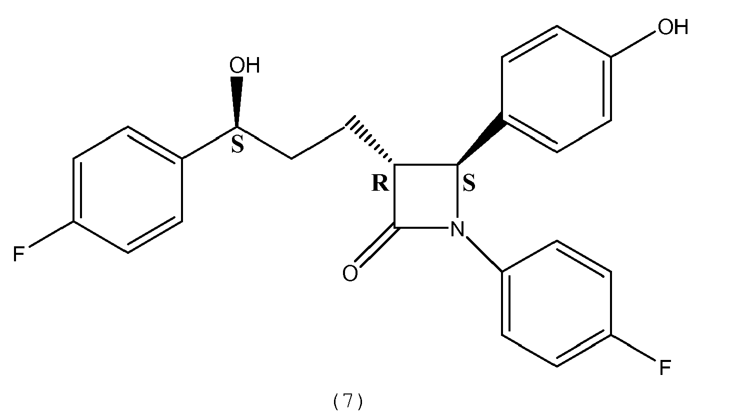

2. The method according to claim 1, wherein the compound of formula (3a) is at a molar ratio with respect to compound (3b) of between 7.5:1 and 8.5:1 in the diastereoisomeric mixture (3a) and (3b) (SRS:SRR) obtained in step (a).

3. The method according to any of the preceding claims, wherein step (b) of hydrolyzing the hydroxyl protecting group is performed between steps (a) and (c).

4. The method according to claim 3, wherein the compound of formula (4a) is at a molar ratio with respect to compound (4b) of between 7.5:1 and 8.5:1 in the diastereoisomeric mixture (4a) and (4b) (SRS:SRR) obtained in step (b).

5. The method according to any of the preceding claims, wherein the silylating agent used in step (d) is N,O-bis-trimethylsilylacetamide.

6. The method according to any of the preceding claims, wherein the fluorine salt used in step (d) is tetrabutylammonium fluoride.

7. The method according to any of the preceding claims, wherein the nitrous acid source of step (f) is NaNO₂ and sulfuric acid; and wherein the alkaline aqueous medium is a sodium bicarbonate solution.

8. The method according to any of the preceding claims, wherein compound (1) is obtained from the compound of formula (8) 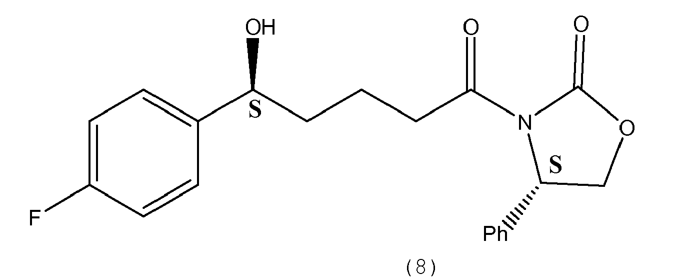 by means of protecting the hydroxyl group with a hydroxyl protecting group.

9. The method according to claim 8, wherein the hydroxyl protecting group (Prot) is trimethylsilyl.

10. A compound of formula (3a): 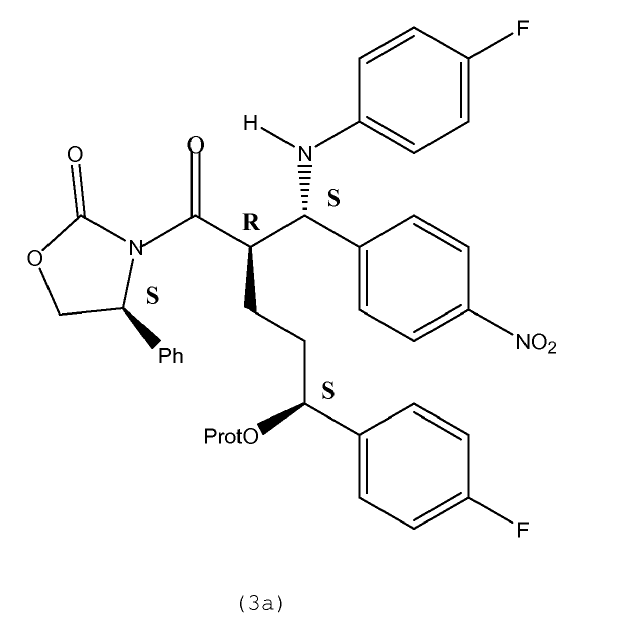 wherein Prot represents a hydroxyl protecting group.

11. A compound of formula (3b): 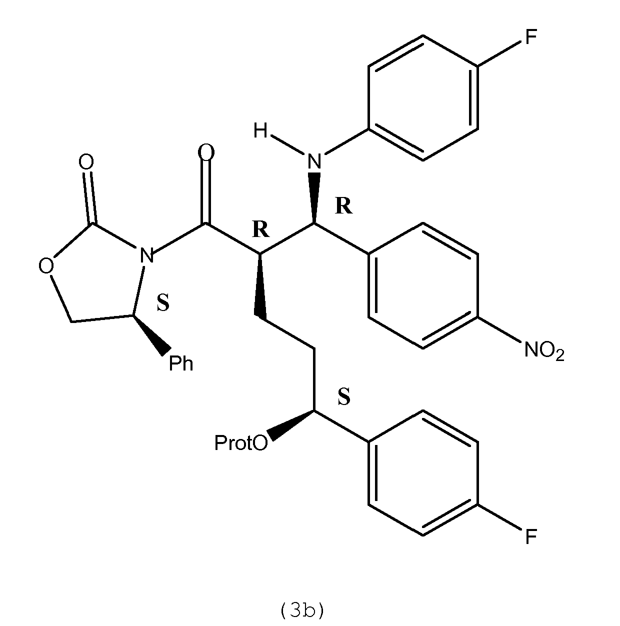 wherein Prot represents a hydroxyl protecting group.

12. A compound of formula (4a):

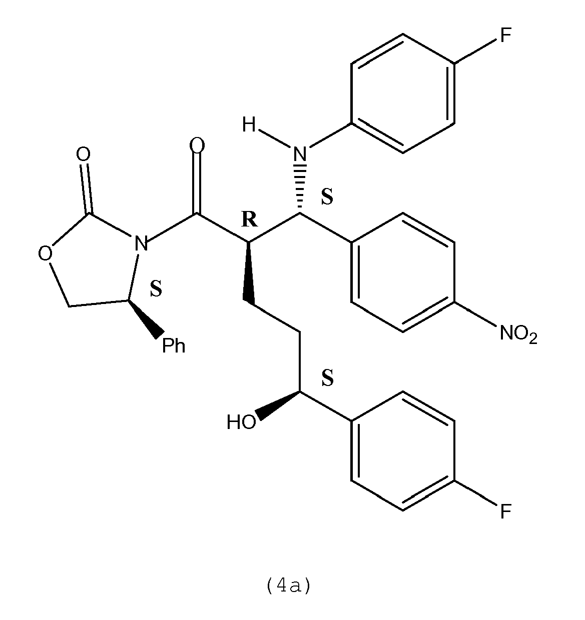

13. A compound of formula (4b):

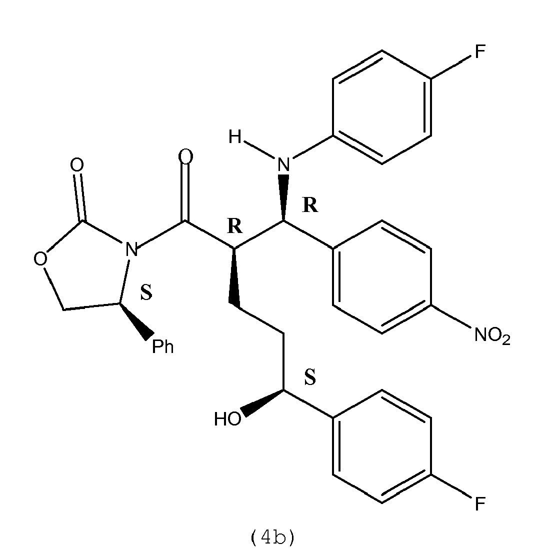

14. A compound of formula (5): 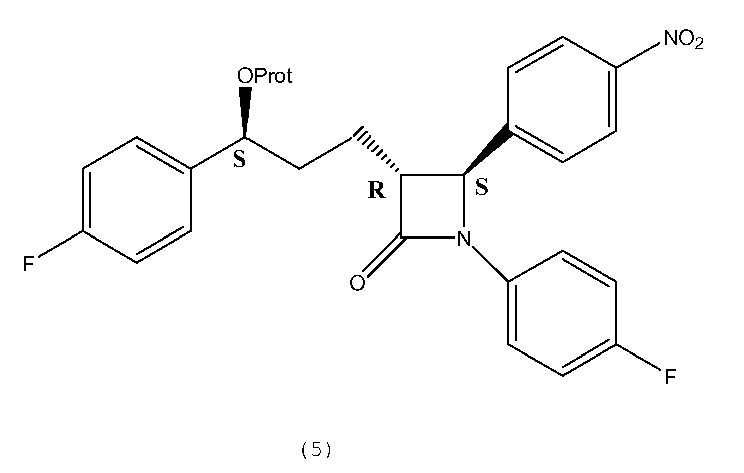 wherein Prot represents a hydroxyl protecting group.

15. A compound of formula (6):

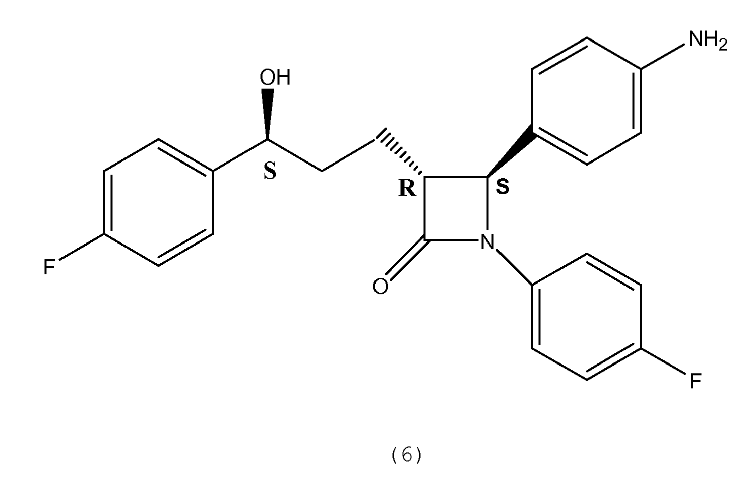

## Patentansprüche

1. Methode zum Herstellen von Ezetimib (7), welche die folgenden Schritte umfasst:
(a) Umsetzen einer Verbindung der Formel (1) 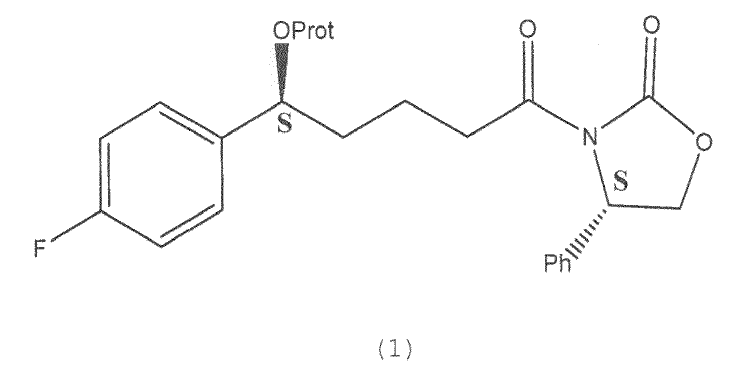 worbei Ph eine Phenylgruppe repräsentiert und Prot eine Hydroxylschutzgruppe repräsentiert,
mit einem Imin der Formel (2) 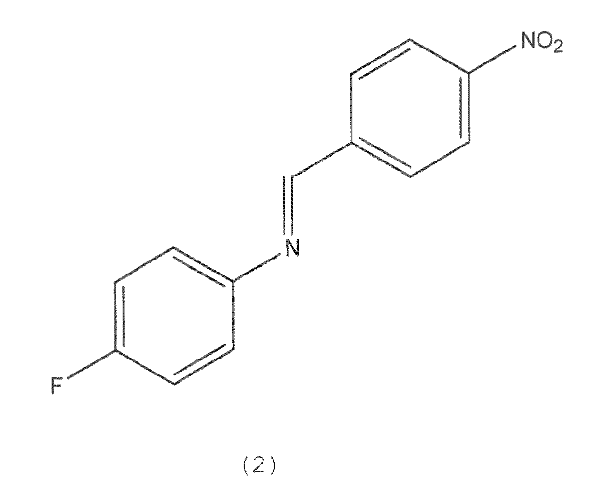 um eine diastereomere Mischung (3a) und (3b) zu ergeben, deren Hauptprodukt die Verbindung der Formel (3a) ist, 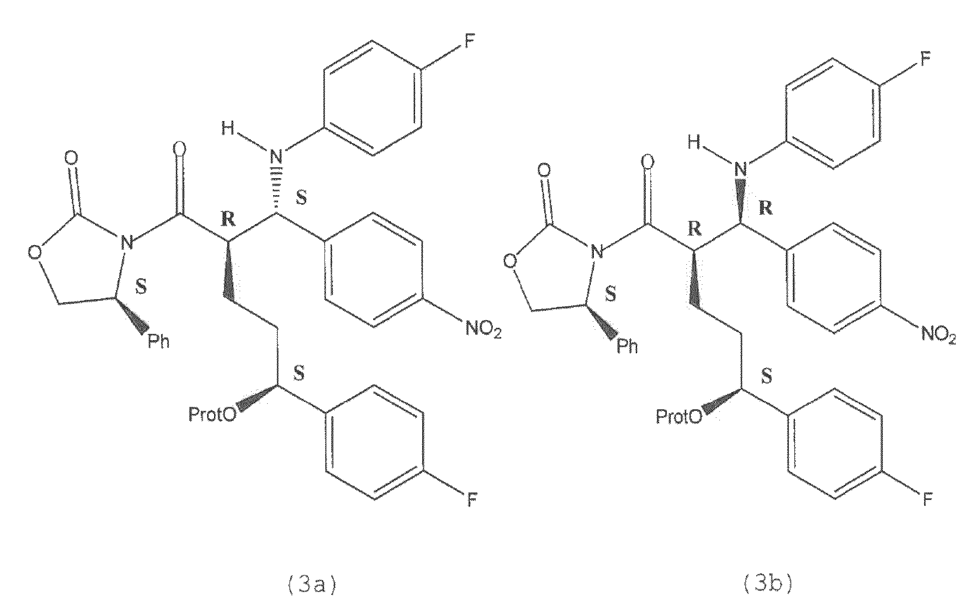
(b) optionales Hydrolysieren der Hydroxylschutzgruppe, um eine diastereomere Mischung (4a) und (4b) zu ergeben, deren Hauptprodukt die Verbindung der Formel (4a) ist, 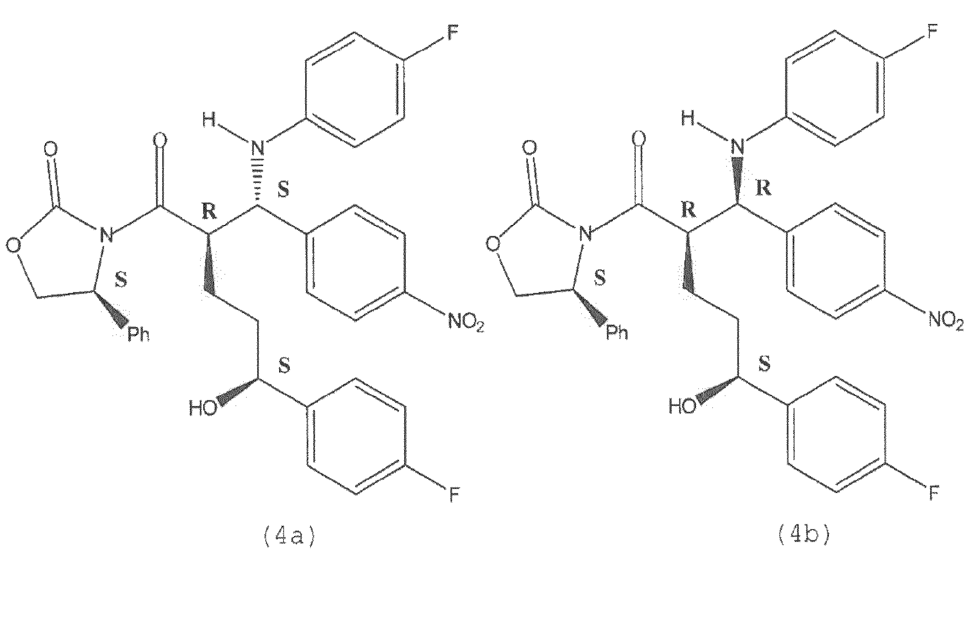
(c) Abtrennen der Verbindung (3a) von der diastereomeren Mischung von Schritt (a) oder, im Fall dass Schritt (b) durchgeführt wurde, Abtrennen der Verbindung (4a) von der diastereomeren Mischung von Schritt (b),
(d) Zyklisieren der Verbindung der Formel (3a) oder der Verbindung der Formel (4a) mit einem silylierenden Reagens und einem Fluorsalz, um Verbindung (5) zu ergeben, 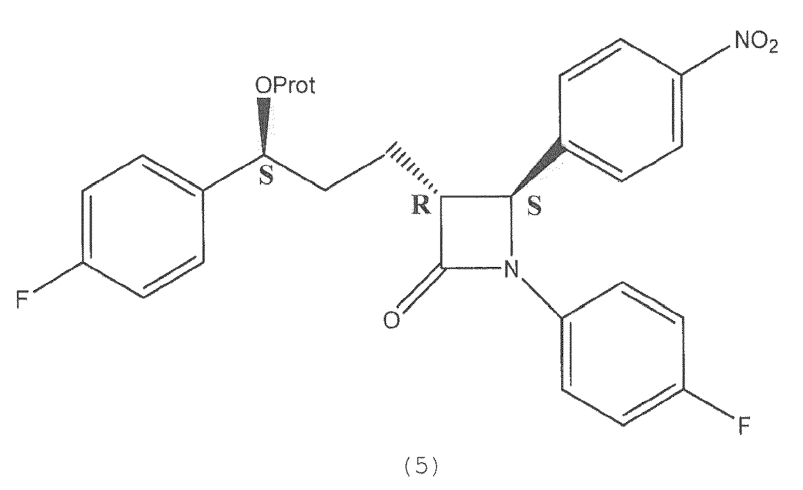
(e) Reduzieren und Entschützen der Verbindung (5), um Verbindung (6) zu ergeben, 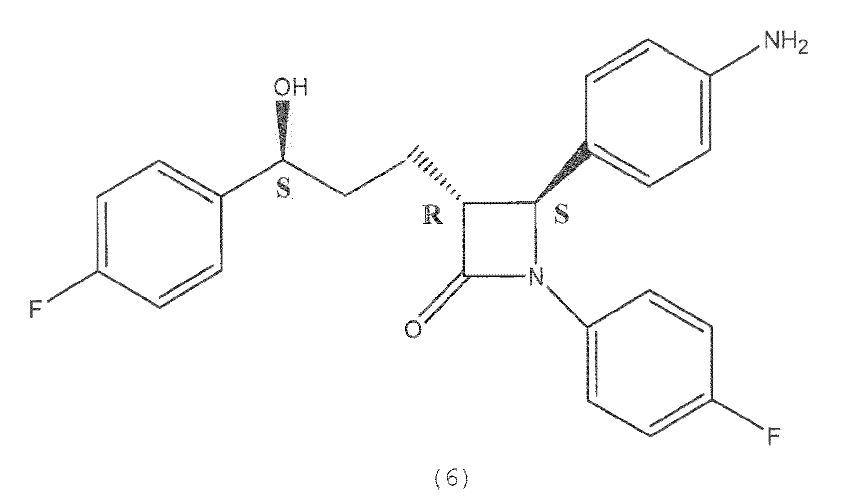
(f) Umwandeln von Verbindung (6) in Ezetimib (7) durch Reaktion mit einer Quelle Salpetriger Säure und anschließender Hydrolyse in alkalischem, wässrigem Medium.

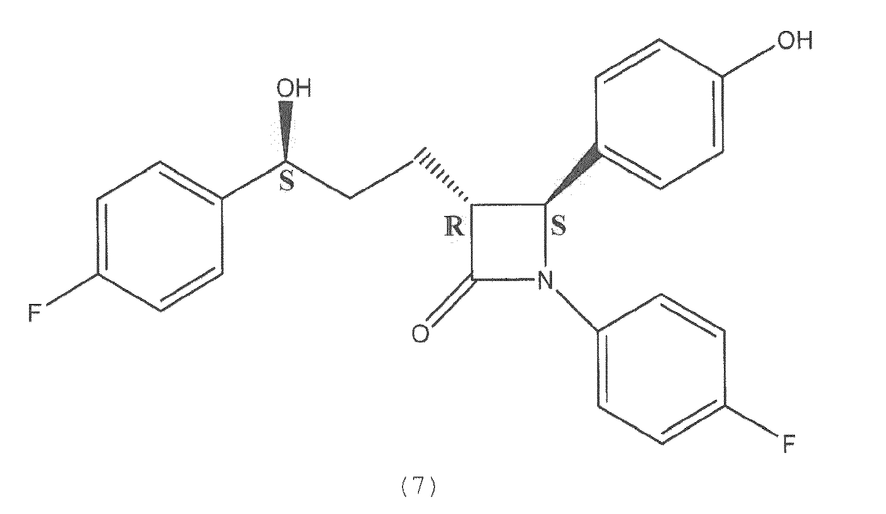

2. Methode gemäß Anspruch 1, wobei die Verbindung der Formel (3a) bei einem molaren Verhältnis in Bezug auf die Verbindung (3b) von zwischen 7,5:1 und 8,5:1 in der diastereomeren Mischung (3a) und (3b) (SRS:SRR), welche in Schritt (a) erhalten wird, ist.

3. Methode gemäß einem der vorangehenden Ansprüche, worin Schritt (b) des Hydrolysierens der Hydroxylschutzgruppe zwischen den Schritten (a) und (c) durchgeführt wird.

4. Methode gemäß Anspruch 3, wobei die Verbindung der Formel (4a) bei einem molaren Verhältnis in Bezug auf die Verbindung der Formel (4b) von zwischen 7,5:1 und 8,5:1 in der diastereomeren Mischung (4a) und (4b) (SRS:SRR), welche in Schritt (b) erhalten wird, ist.

5. Methode gemäß einem der vorangehenden Ansprüche, worin das silylierende Reagens, welches in Schritt (d) eingesetzt wird, N,O-Bis-trimethylsilylacetamid, ist.

6. Methode gemäß einem der vorangehenden Ansprüche, wobei das Fluorsalz, welches in Schritt (d) eingesetzt wird, Tetrabutylammoniumfluorid, ist.

7. Methode gemäß einem der vorangehenden Ansprüche, wobei die Quelle Salpetriger Säure von Schritt (f) NaNO₂ und Schwefelsäure ist; und worin das alkalische, wässrige Medium eine Natriumhydrogenkarbonatlösung ist.

8. Methode gemäß einem der vorangehenden Ansprüche, wobei Verbindung (1) aus der Verbindung der Formel (8) 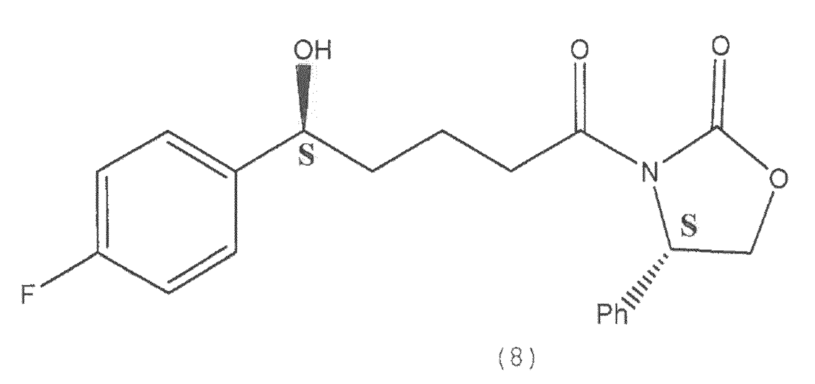 durch Mittel des Schützens der Hydroxylgruppe mit einer Hydroxylschutzgruppe erhalten wird.

9. Methode gemäß Anspruch 8, wobei die Hydroxylschutzgruppe (Prot) Trimethylsilyl ist.

10. Verbindung der Formel (3a): 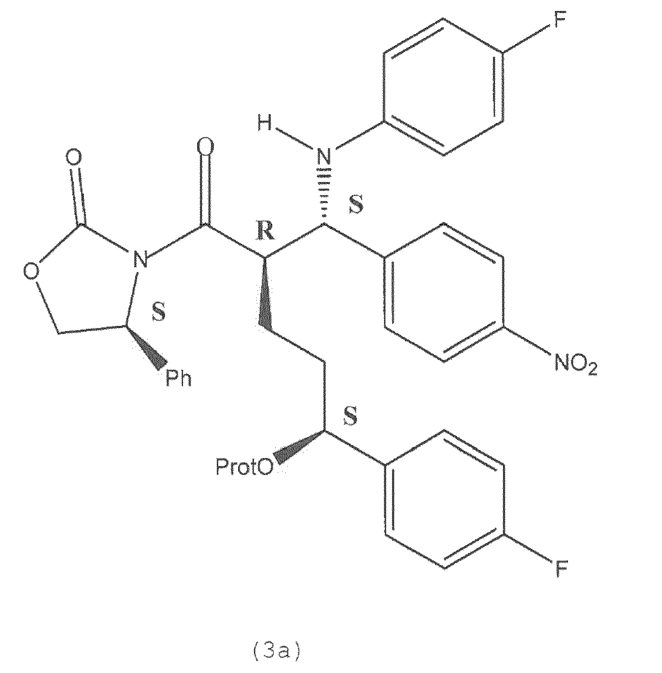 worin Prot eine Hydroxylschutzgruppe repräsentiert.

11. Verbindung der Formel (3b): 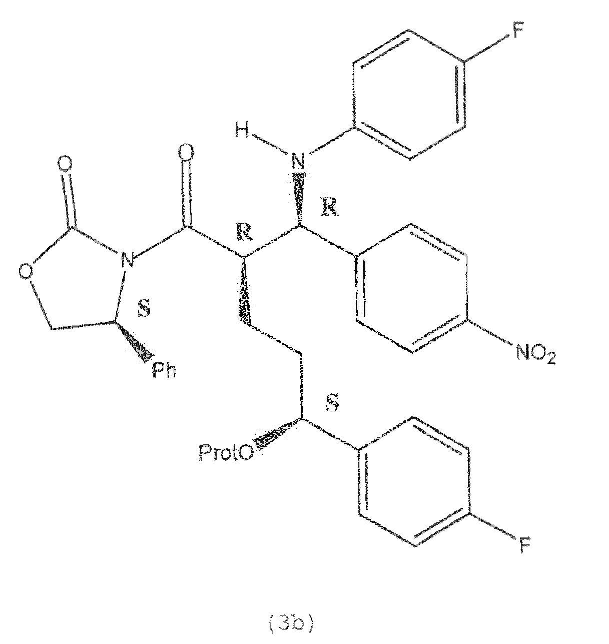 worin Prot eine Hydroxylschutzgruppe repräsentiert.

12. Verbindung der Formel (4a):

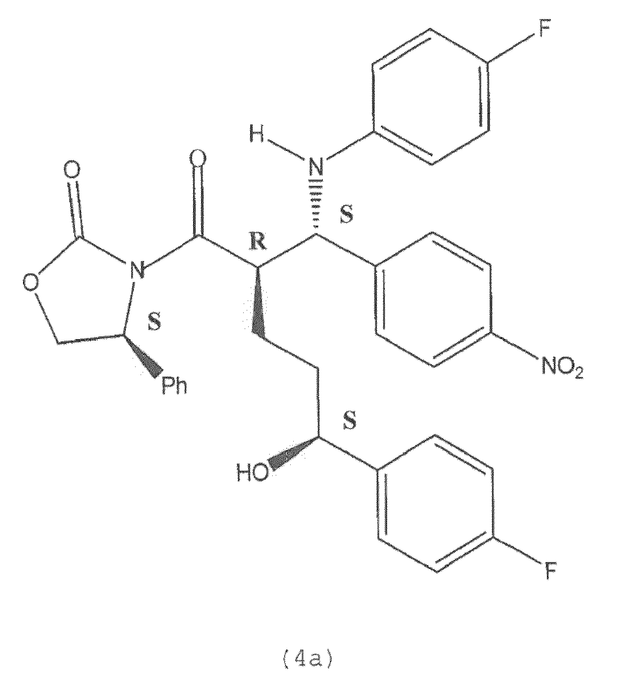

13. Verbindung der Formel (4b):

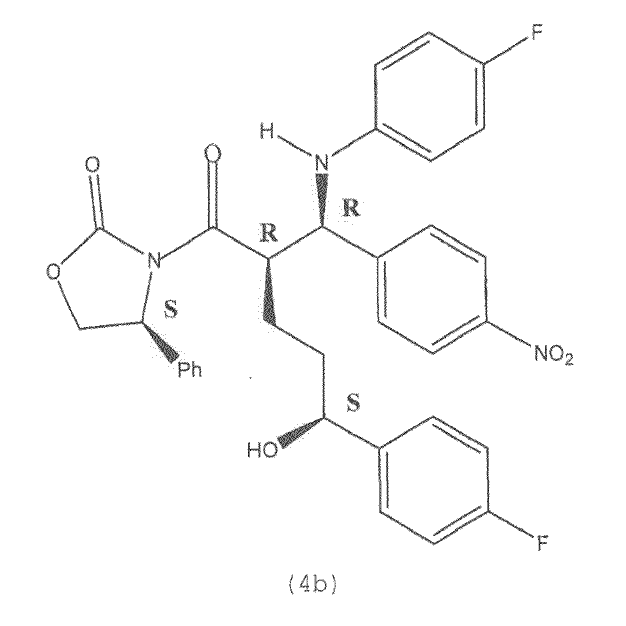

14. Verbindung der Formel (5): 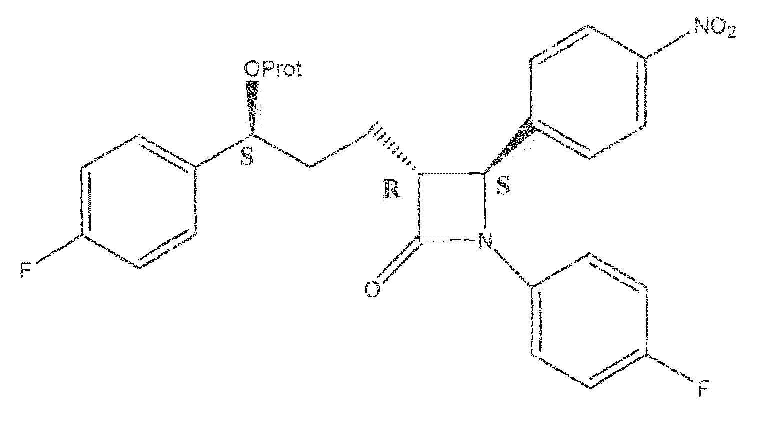 worin Prot eine Hydroxylschutzgruppe repräsentiert.

15. Verbindung der Formel (6):

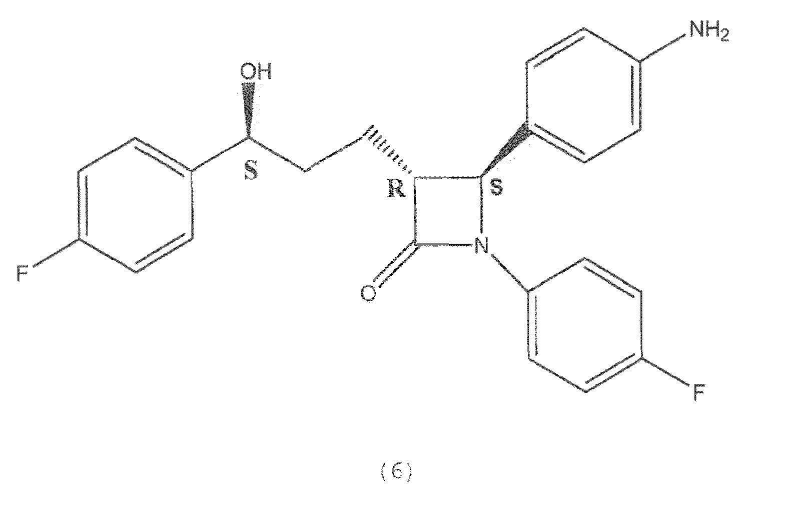

## Revendications

1. Procédé de préparation de l'ézétimibe (7), comprenant les étapes suivantes :
(a) réaction d'un composé de formule (1) 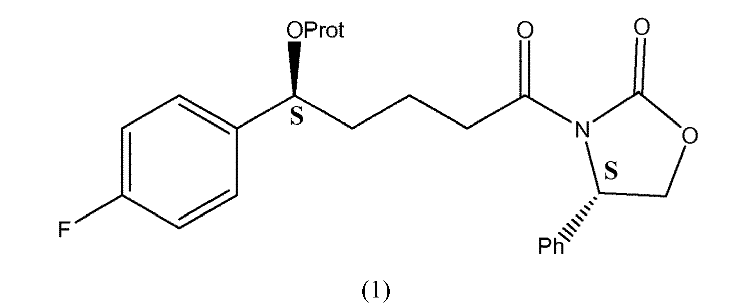 dans laquelle Ph représente un groupe phényle et Prot représente un groupe protecteur d'hydroxyle,
avec une imine de formule (2) 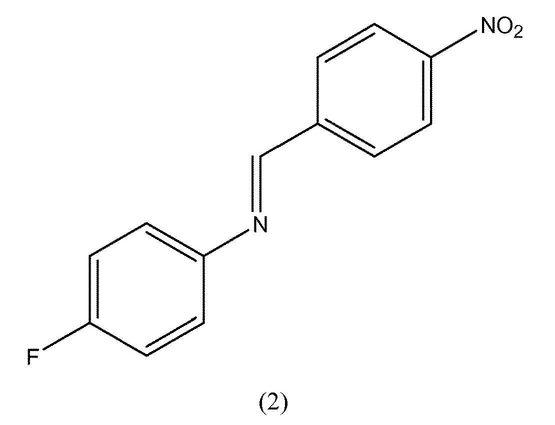 pour engendrer un mélange de diastéréoisomères (3a) et (3b), dont le produit principal est le composé de formule (3a), 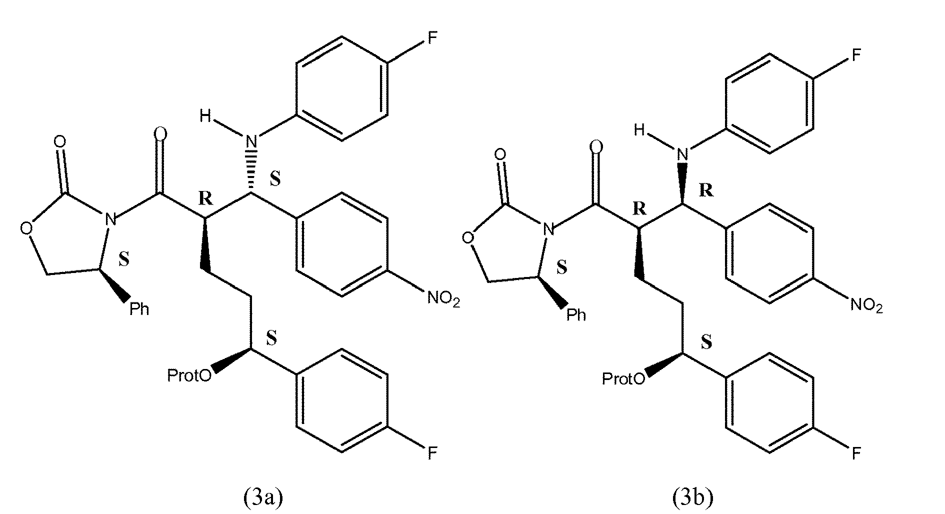
(b) éventuellement l'hydrolyse du groupe protecteur d'hydroxyle pour engendrer un mélange de diastéréoisomères (4a) et (4b), dont le produit principal est le composé de formule (4a), 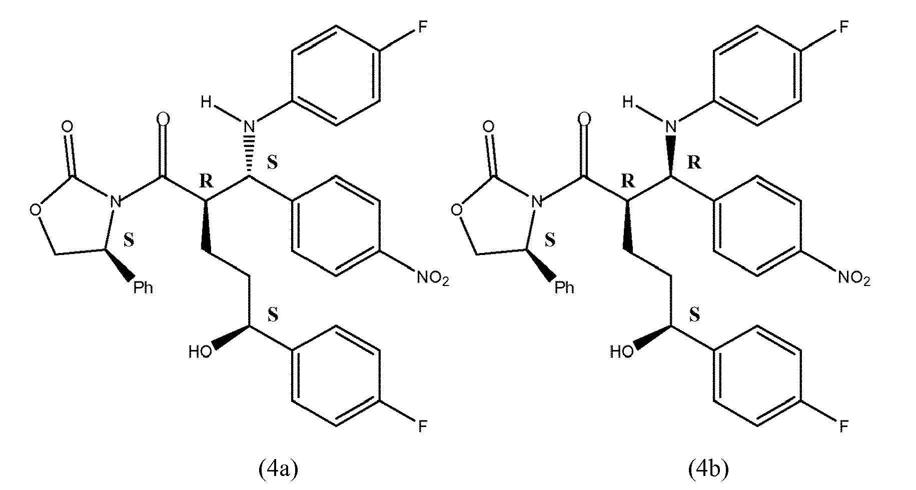
(c) la séparation du composé (3a) à partir du mélange de diastéréoisomères de l'étape (a) ou, dans le cas où l'étape (b) a été effectuée, la séparation du composé (4a) à partir du mélange de diastéréoisomères de l'étape (b),
(d) la cyclisation du composé de formule (3a) ou du composé de formule (4a) avec un agent de silylation et un sel de fluor pour engendrer le composé (5), 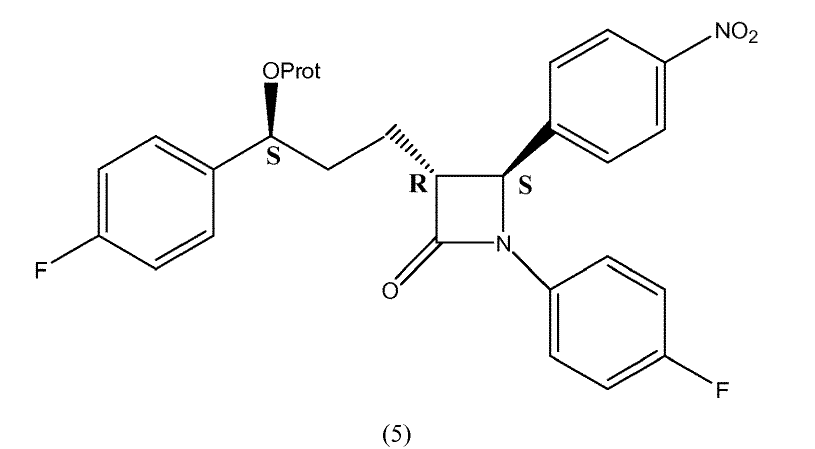
(e) la réduction et la déprotection du composé (5) pour engendrer le composé (6), 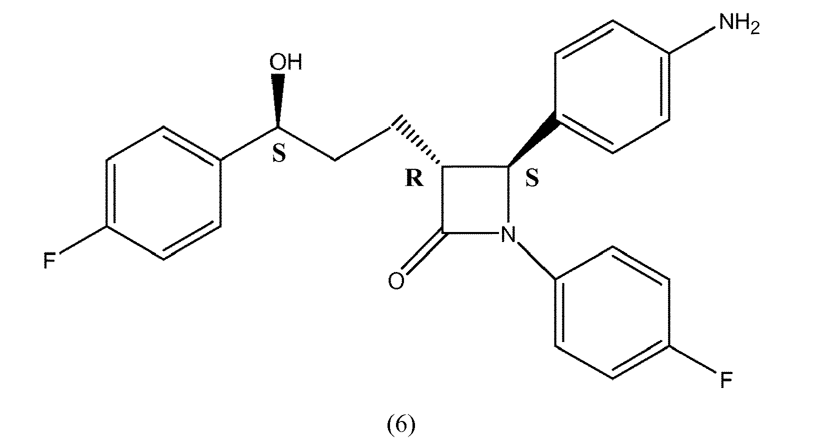
(f) la conversion du composé (6) en ézétimibe (7) par réaction avec une source d'acide nitreux et hydrolyse subséquente dans un milieu aqueux alcalin.

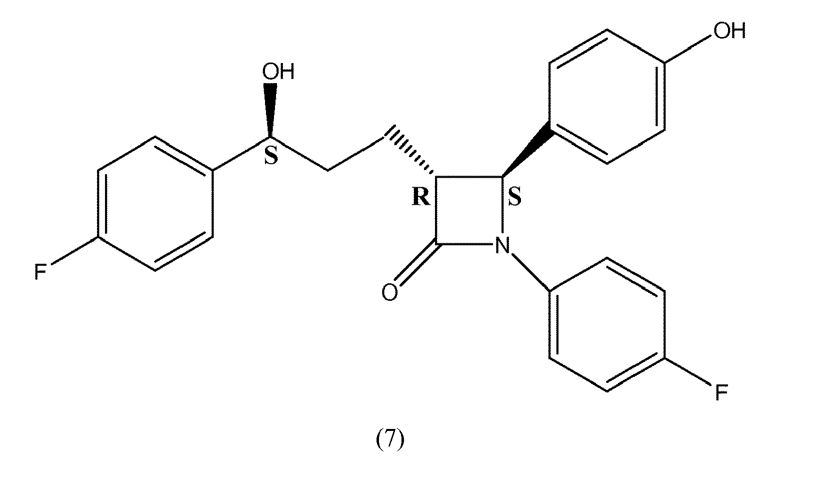

2. Procédé selon la revendication 1, dans lequel le composé de formule (3a) est présent en un rapport molaire, par rapport au composé (3b), compris entre 7,5:1 et 8,5:1 dans le mélange de diastéréoisomères (3a) et (3b) (SRS/SRR) obtenu dans l'étape (a).

3. Procédé selon l'une quelconque des revendications précédentes, dans lequel l'étape (b) d'hydrolyse du groupe protecteur d'hydroxyle est effectuée entre les étapes (a) et (c).

4. Procédé selon la revendication 3, dans lequel le composé de formule (4a) est présent en un rapport molaire, par rapport au composé (4b), compris entre 7,5:1 et 8,5:1 dans le mélange de diastéréoisomères (4a) et (4b) (SRS/SRR) obtenu dans l'étape (b).

5. Procédé selon l'une quelconque des revendications précédentes, dans lequel l'agent de silylation utilisé dans l'étape (d) est le N,O-bis-triméthylsilylacétamide.

6. Procédé selon l'une quelconque des revendications précédentes, dans lequel le sel de fluor utilisé dans l'étape (d) est le fluorure de tétrabutylammonium.

7. Procédé selon l'une quelconque des revendications précédentes, dans lequel la source d'acide nitreux de l'étape (f) est NaNO₂ et l'acide sulfurique ; et dans lequel le milieu aqueux alcalin est une solution de bicarbonate de sodium.

8. Procédé selon l'une quelconque des revendications précédentes, dans lequel le composé (1) est obtenu à partir du composé de formule (8) 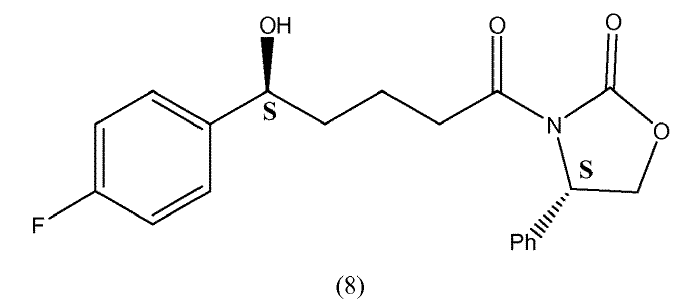 au moyen d'une protection du groupe hydroxyle avec un groupe protecteur d'hydroxyle.

9. Procédé selon la revendication 8, dans lequel le groupe protecteur d'hydroxyle (Prot) est triméthylsilyle.

10. Composé de formule (3a) : 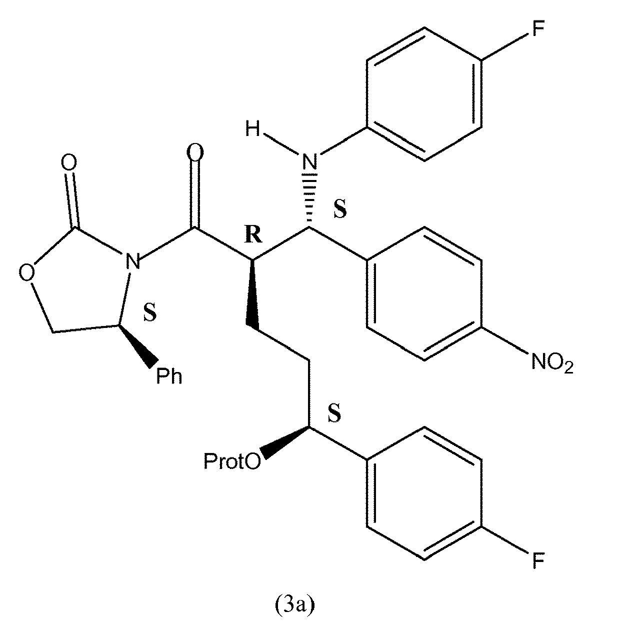 dans laquelle Prot représente un groupe protecteur d'hydroxyle.

11. Composé de formule (3b) : 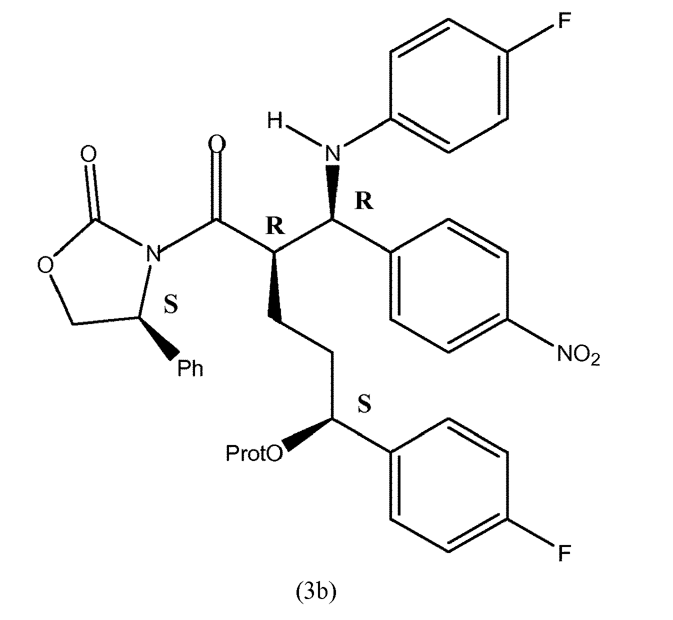 dans laquelle Prot représente un groupe protecteur d'hydroxyle.

12. Composé de formule (4a) :

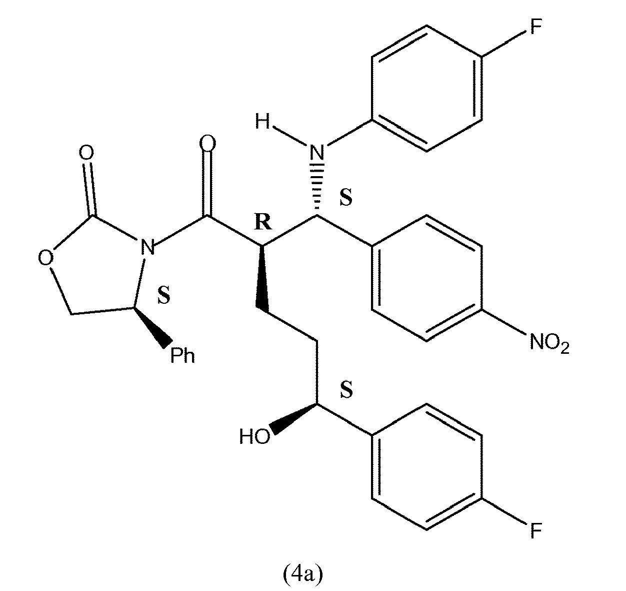

13. Composé de formule (4b) :

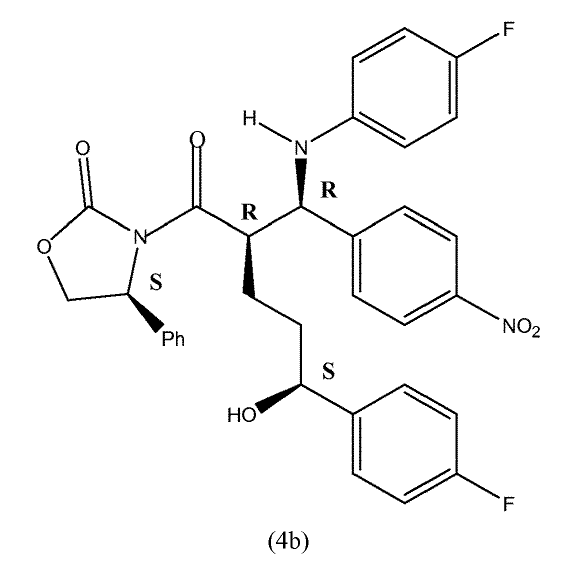

14. Composé de formule (5) : 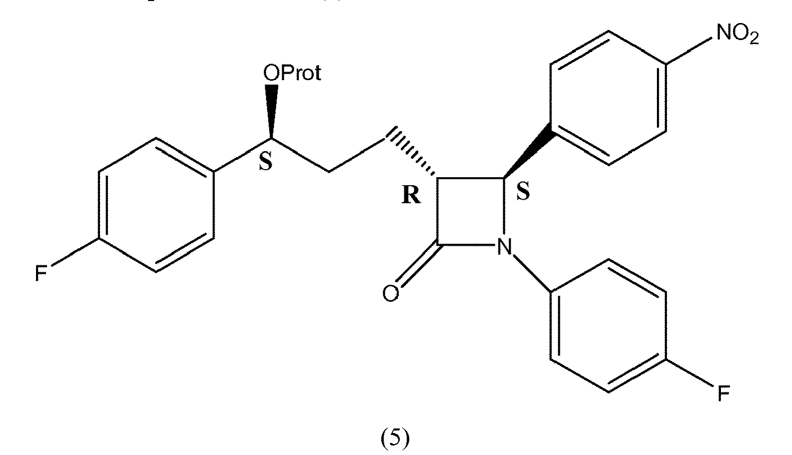 dans laquelle Prot représente un groupe protecteur d'hydroxyle.

15. Composé de formule (6) :
